# EUROPEAN PATENT APPLICATION

(11) **EP 2 546 355 A1**
(43) Date of publication of application: **16.01.2013**
(21) Application number: 12176993.9
(22) Date of filing: 14.03.2008
(51) Int. Cl.: C12Q 1/48

(54) **Methods for identifying diabetes and obesity therapeutics**

(30) Priority: 14.03.2007 US 906922 P
(62) Divisional of application: 08742098.0
(71) Applicant: Washington University, St. Louis, MO 63130 (US); Beth Israel Deaconess Medical Center, Inc., Boston, MA 02215 (US)
(72) Inventor: Hurov, Jonathan, B., Boston, MA 02072 (US); Piwnica-Worms, Helen, St Louis, MO 63110-1093 (US); Cantley, Lewis C., Cambridge, MA 02138 (US)
(74) Representative: White, Nina Louise

(57) **Abstract**

The invention relates to inhibition of Par-1b kinase activity for treating disorders including diabetes and obesity. The invention also relates to screening for compounds or compositions that inhibit the kinase activity of Par-1b protein, which compounds and compositions are useful in the treatment of diabetes and obesity, as well as preparing compounds for treatment of diabetes and obesity.

Sequence listing
SEQUENCE LISTING

<110>  WASHINGTON, UNIVERSITY
       BETH ISRAEL DEACONESS MEDICAL CENTER, INC.
       HUROV, Jonathan B.
       PIWNICA-WORMS, Helen
       CANTLEY, Lewis C.

<120>  METHODS FOR IDENTIFYING DIABETES AND OBESITY THERAPEUTICS

<130>  B0662.70072WO00

<150>  60/906922
<151>  2007-03-14

<160>  1

<170>  PatentIn version 3.4

<210>  1
<211>  21
<212>  RNA
<213>  Artificial

<220>
<223>  siRNA sequence

<400>  1
aagacucaac ugaacuccuc c                                                21

## Description

### Government Support

This invention was made in part with government support under grant numbers P50 CA94056, RO1 GM56203 and R01 DK40936 from the National Institutes of Health (NIH). The government has certain rights in this invention.

### Field of the Invention

The invention relates to inhibition of Par-1b activity for treating disorders including diabetes and obesity. The invention also relates to screening for agents that inhibit the activities of Par-1b protein, which are useful in the treatment of diabetes and obesity, as well as preparing compounds for treatment of diabetes and obesity.

### Background of the Invention

Insulin resistance and obesity are two physiological perturbations central to the development of Type 2 diabetes. An estimated 15.7 million Americans have diabetes, and individuals with adult-onset, type 2, diabetes represent 90 to 95 percent of all diabetics. Almost one-third of all diabetics in the U.S. are unaware that they have the disorder, and undetected and uncontrolled diabetes can have serious side effects, such as blindness, heart disease, nerve disease, and kidney disease. In addition to the increased clinical risks, type 2 diabetes may also result in a reduced quality of life for the affected individual.

Obesity also is a condition that is on the increase throughout the world. In the developed world, 2 to 7% of total health care costs are attributable to obesity (Hossain et al., New Engl J Med. 2007 Jan 18. 356:213-215). In the United States alone, the combined direct and indirect costs of obesity were estimated to be $123 billion in 2001 (Hossain et al., 2007). Obesity affects the health of humans in several ways; most prominently it contributes to an increase in cases of diabetes and hypertension. According to some reports, approximately 90% of type 2 diabetes is the result of excess weight (Hossain et al., 2007).

Because type 2 diabetes and obesity are major disorders in current society, which have serious health and life quality consequences, improved methods of treatment are needed to provide better therapy of diabetes and obesity with fewer and/or lesser side effects.

### Summary of the Invention

The identification and characterization of genes involved in regulation of insulin sensitivity and glucose uptake is key to the design and development of new drug therapies for type 2 diabetes and obesity. In this study we show that the polarity kinase Par-1b/MARK2 is required for regulating glucose metabolism *in vivo.* Mice null for Par-1b are lean, insulin hypersensitive, resistant to high fat diet-induced weight gain and are hypermetabolic. ¹⁸F-FDG microPET and hyperinsulinemic-euglycemic clamp analyses demonstrate increased glucose uptake into white and brown adipose tissue, but not into skeletal muscle of Par-1b null mice relative to wild-type controls. Taken together, these data indicate that Par-1b is a novel regulator of glucose metabolism and adiposity in the whole animal and therefore is a valuable drug target for the treatment of both Type 2 diabetes and obesity.

According to one aspect of the invention, methods for identifying compounds or compositions useful as pharmacological agents for the treatment of diabetes or obesity are provided. The methods include contacting a Par-1b polypeptide with a candidate pharmacological agent, and determining the kinase activity of the contacted Par-1b polypeptide. A decrease in the kinase activity of the Par-1b polypeptide contacted with the candidate pharmacological agent relative to a control amount of kinase activity of the Par-1b polypeptide is an indication that the candidate pharmacological agent is useful in the treatment of diabetes or obesity.

In some embodiments, the methods also include determining a second amount of kinase activity of the Par-1b polypeptide in the absence of the candidate pharmacological agent, and using the second amount of activity of the Par-1b polypeptide as the control amount of activity.

In certain preferred embodiments, the kinase activity of the Par-1b polypeptide is measured by phosphorylation of a tau, Dvl, Par-3, Cdc25C, MAP2 or MAP4 protein or a peptide comprising the phosphorylation site of a tau, Dvl, Par-3, Cdc25C, MAP2 or MAP4 protein. Preferably the phosphorylation is measured by a phospho-specific antibody or an antigen-binding fragment thereof.

In other preferred embodiments, the Par-1b polypeptide is contained within a cell, and the cell is contacted with the candidate pharmacological agent.

According to another aspect of the invention, methods for treating a subject having or suspected of having disorder characterized by reduced insulin responsiveness or obesity are provided. The methods include administering to a subject in need of such treatment an effective amount of a compound or composition that decreases the kinase activity of Par-1b polypeptide in the subject, as a treatment for the disorder.

In some embodiments, the disorder characterized by reduced insulin responsiveness is type 2 diabetes.

In other embodiments, the compound or composition decreases the amount of Par-1b polypeptide. Preferably the compound or composition that decreases the amount of Par-1b polypeptide is a siRNA/RNAi molecule or an antisense nucleic acid molecule; more preferably the siRNA/RNAi molecule comprises the nucleotide sequence AAG ACU CAA CUG AAC UCC UCC (SEQ ID NO:1).

According to another aspect of the invention, methods for preparing a drug for the treatment of a disorder characterized by reduced insulin responsiveness or obesity are provided. The methods include identifying a compound or composition that decreases kinase activity of a Par-1b polypeptide and formulating the compound or composition for administration to a subject in need of such treatment.

In some embodiments, the disorder characterized by reduced insulin responsiveness is type 2 diabetes.

In other embodiments, the compound or composition that decreases kinase activity of a Par-1b polypeptide is identified by any of the foregoing methods for identifying compounds or compositions useful as pharmacological agents for the treatment of diabetes or obesity.

In another aspect, the invention provides for use of the foregoing agents, compounds and molecules in the preparation of medicaments, particularly medicaments for the treatment of reduced insulin responsiveness, e.g., type 2 diabetes, and obesity.

These and other aspects of the invention are described further below.

### Brief Description of the Drawings

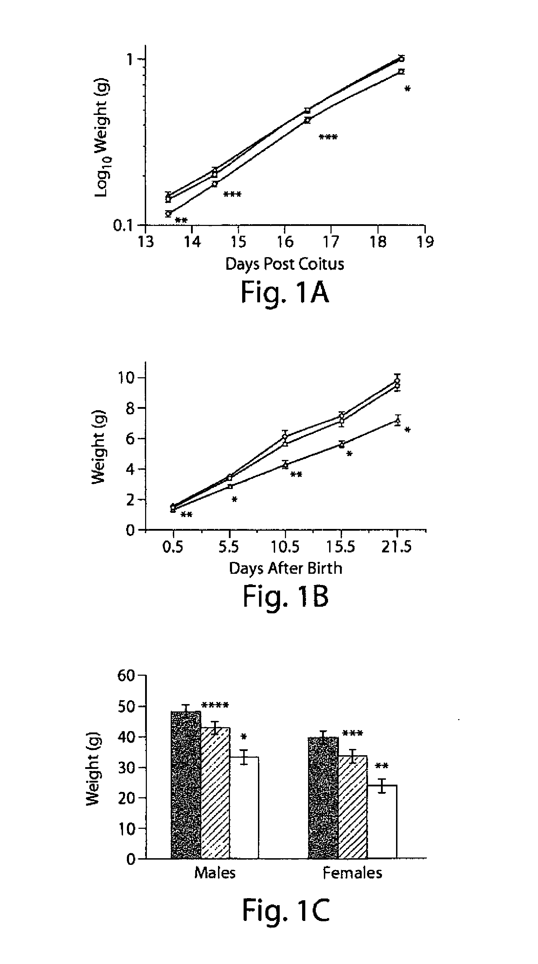

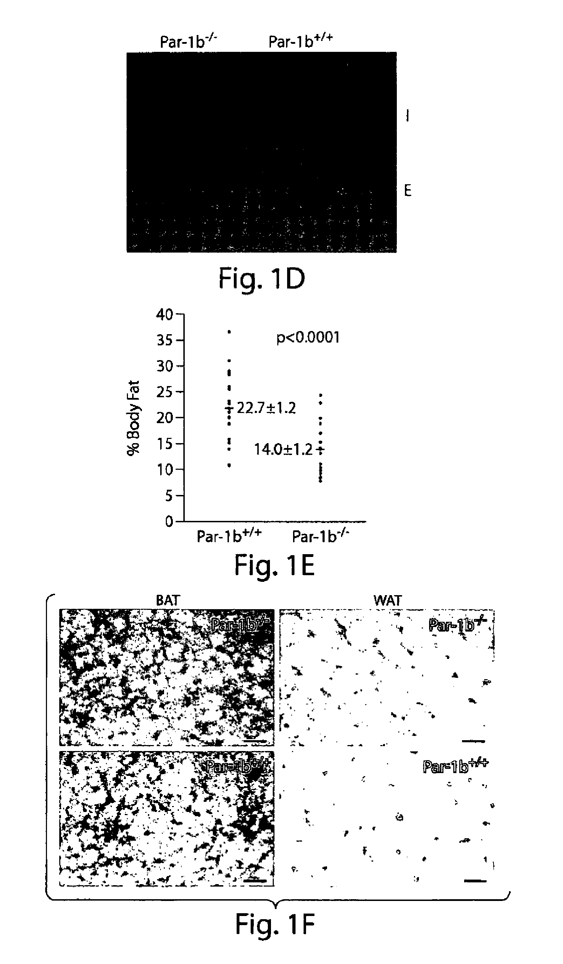

**Figure 1****. Par-1b null mice are growth retarded and have disproportionate reductions in adiposity.**
   **(A)** Body weights of embryos from 13.5 to 18.5 days post coitus (d.p.c.) (n = 5-13 per genotype per time point). **(B)** Body weights of mice from birth to 3 weeks of age (n= 4-19 per genotype per time point). **(C)** Body weights of Par-1b wild-type (Par-1b^{+/+}, black bars), heterozygous (Par-1b^{+/-}, red bars) or null (Par-1b^{-/-}, grey bars) mice at one year of age (n= 7-13 per genotype per sex). **(D)** Comparison of Par-1b wild-type and null inguinal (I) and epidydymal (E) fat pads. **(E)** Scatter plot depiction of adiposity in 10-12 week old male Par-1b wild-type and null mice expressed as a ratio of fat mass to body mass as determined by ¹H-MRS. Individual mice for each genotype are shown with averages and p-value indicated (n=18-20 per genotype). **(F)** Hematoxylin and eosin stain of brown and white adipose tissue (BAT and WAT) from Par-1b wild-type and null mice. All values are presented as the averages ± standard error. Student's t test was performed for comparisons between groups. p value designations are as follows: **** (p<0.05), ***(p<0.01), ** (p<0.005), *(p<0.001).

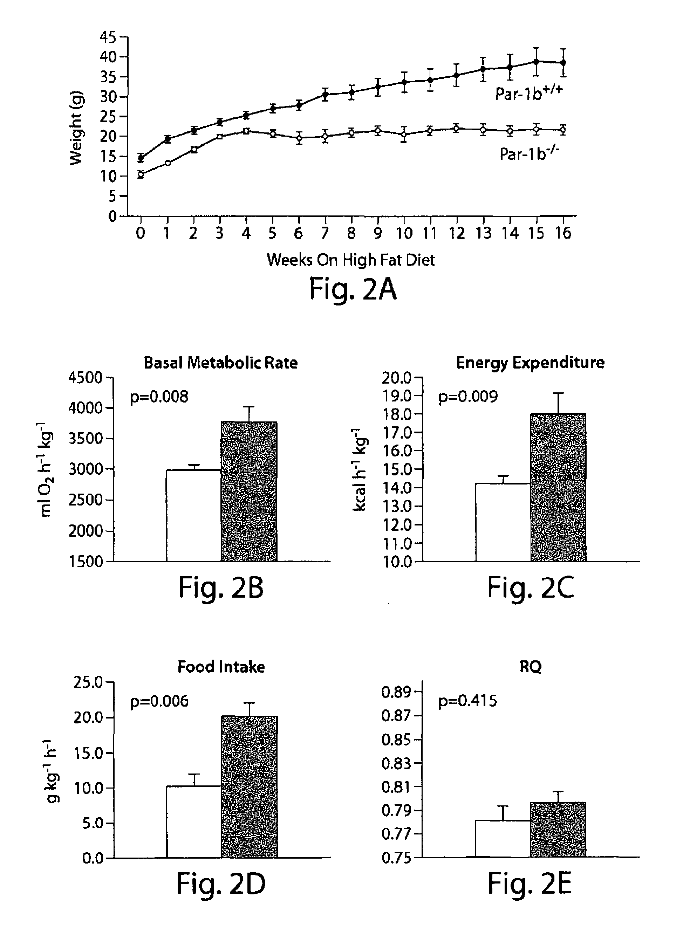

**Figure 2****. Par-1b null mice are resistant to weight gain and are hypermetabolic on a high fat diet.**
   Par-1b wild-type and null males (n=5 per genotype) were fed a high fat diet for 16 weeks starting at 3 weeks of age. **(A)** Body weight of Par-1b wild-type and null mice during the high fat diet. Averages are plotted ± standard error. All data points represent statistical differences between wild-type and null mice with p<0.05. In an independent experiment, male Par-1b null (purple bars) and wild-type mice (empty bars) (n=8-12 per genotype) were fed a high fat diet for 8 weeks followed by analysis. Par-1b null mice exhibit increased metabolic rate **(B),** energy expenditure **(C),** and food intake **(D),** on a high fat diet. Respiratory quotient (VCO₂/VO₂) of Par-1b null mice is not statistically different than wild-type controls **(E).** Student t-test p values are indicated in panel.

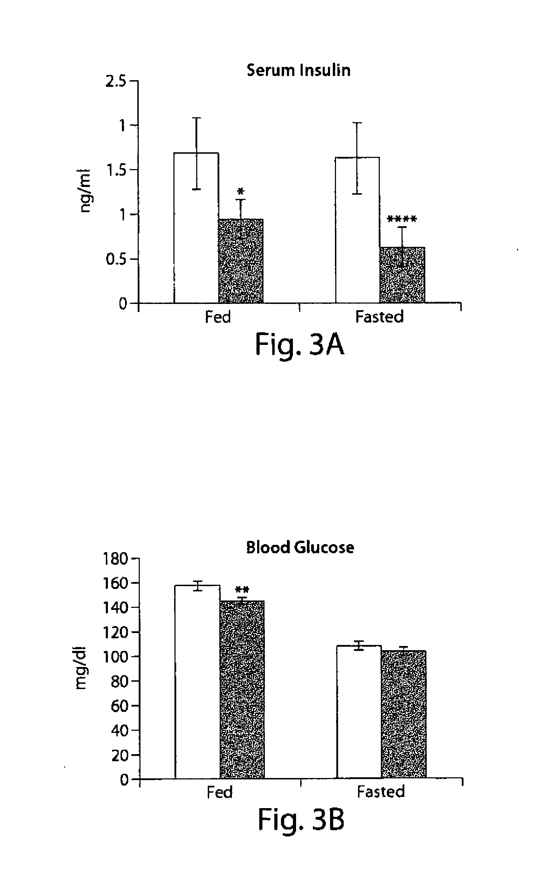

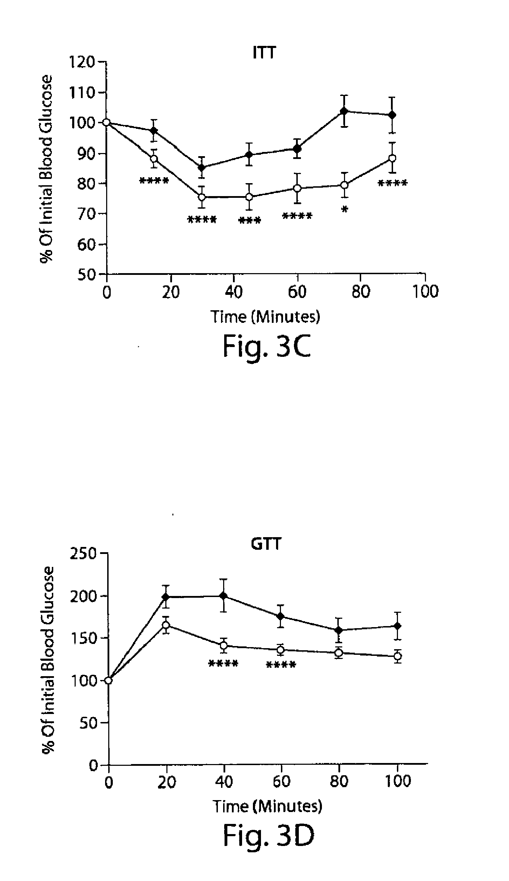

**Figure 3****. Enhanced insulin sensitivity and glucose tolerance in Par-1b null mice.**
   **(A)** Retro-orbital bleeds were obtained from fed or fasted (12 hours) Par-1b^{+/+} (empty bars) and Par-1b^{-/-} (purple bars) mice and serum insulin levels were measured by radioimmunoassay (n=18 mice per genotype) **(B)** Blood glucose levels were determined for fed or fasted mice (n=18 mice per genotype). **(C)** Insulin tolerance tests (ITT) were performed by intraperitoneal injection of 0.30 IU/kg insulin into Par-1b^{+/+} (black line, diamonds) and Par-1b^{-/-} (purple line, circles) littermates (n=14 mice per genotype). Tail bleeds were obtained and glucose levels were monitored at 15-minute intervals after insulin injection. Data is plotted as % blood glucose at time 0 prior to injection. (D) Glucose tolerance tests were performed by intrperitoneal injection of D-glucose at 1 mg/g body weight into Par-1b^{+/+} and Par-1b^{-/-} littermates (n=10 mice per genotype). Tail bleeds were obtained and glucose levels were monitored at 20-minute intervals after glucose injection. Data is plotted as % blood glucose at time 0 prior to injection. Standard error is plotted on the y-axis for all values in panels A-D. Student's t test was performed for comparisons between two groups. p values were as follows: **** (p<0.05), ***(p<0.01), ** (p<0.005), *(p<0.001).
**Figure 4****. Micro positron emission tomography (microPET) analysis of ¹⁸F-FDG uptake in Par-1b^{+/+} and Par-1b^{-/-} mice.**
   Representative coronal section image of Par-1b^{+/+} and Par-1b^{-/-} mice 1 hour after ¹⁸F-FDG injection. Par-1b^{-/-} exhibit consistently elevated levels of uptake in intrascapular brown fat (BAT) pads.
**Figure 5****. Resistance to hepatic steatosis in Par-1b-/- mice.**
   **(A)** and **(B).** Low power view of hematoxylin and eosin-stained liver sections show steatosis with accentuation of lobules in the wild-type, whereas sections from null mice show normal liver parenchyma (scale bars: 1mm). **(C)** The distribution of fat within the hepatic lobule (dashed line) is mainly centrilobular; within zone 3 of the hepatic acinus (straight lines). (scale bars: 500 mm). **(D)** The centrilobular steatosis is absent in sections from the null mice (scale bars: 500 mm). **(E)** and **(F).** Oil red **O** staining of fresh-frozen liver tissue from wild-type (E) and KO mice (F), respectively. The insets show micro and macrovesicles in the wild-type while only minimal reactivity is seen in F.

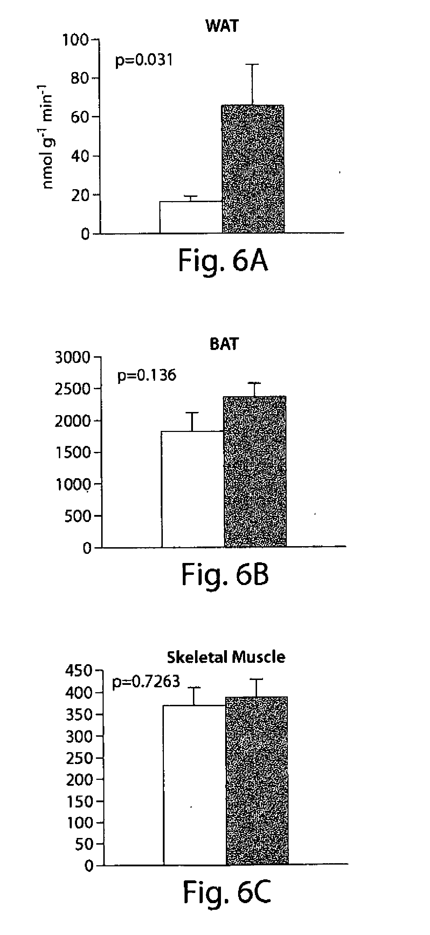

**Figure 6****. Peripheral insulin hypersensitivity in Par-1b null mice due to increased glucose uptake into adipose tissue.**
   Hyperinsulinemic-euglycemic clamp experiments were followed by quantitation of radiolabeled glucose uptake into white adipose tissue **(A),** brown adipose tissue **(B)** and skeletal muscle (gastrocnemius) **(C)** from Par-1b^{+/+} (white bars) and Par-1b^{-/-} (purple bars) mice. Averages are plotted ± standard errors, using n=8-9 mice per genotype.
**Figure 7****.**
   Graphical analysis of ¹⁸F-FDG uptake in intrascapular brown fat pads, skeletal muscle, brain and heart of Par-1b^{+/+} (open bar, n = 4) and Par-1b^{-/-} (solid bar, n = 4) mice 1 hour after ¹⁸F-FDG injection and under the conditions shown. Data for ¹⁸F-FDG uptake are expressed as mean standard uptake values (SUV). Error bars are standard error of the mean (SEM).

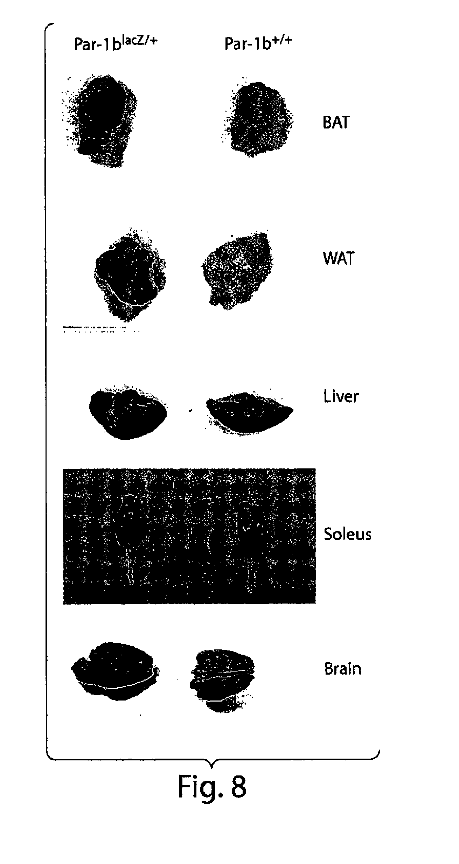

**Figure 8****. Par-1b is expressed in BAT, WAT, liver and brain, but not skeletal muscle.**
   Tissues were analyzed for β-galactosidase activity using mice that carry a β-galactosidase cassette whose expression is driven by the endogenous Par-1b promoter.

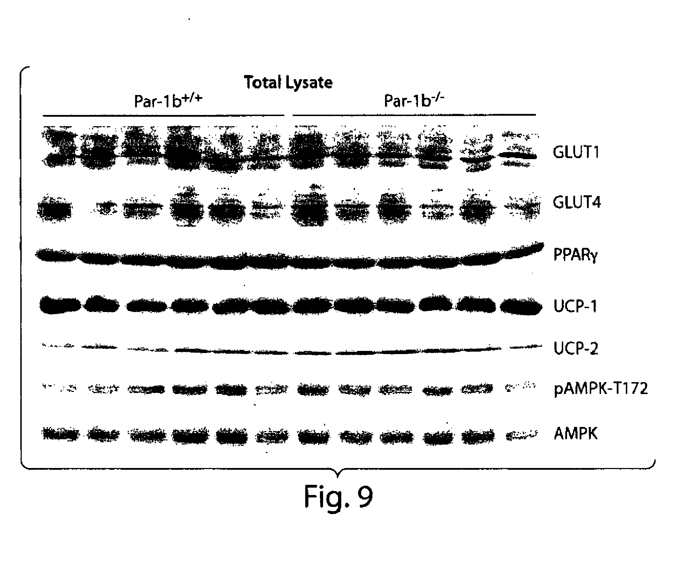

**Figure 9****. Par-1b deficiency does not lead to changes in the expression levels of PPARγ, UCP1, UCP2, GLUT1, GLUT4 in brown adipose tissue.**
   Brown adipose tissue was homogenized and 50 µg of protein from whole cell extracts were analyzed by immunoblotting with the indicated antibodies.

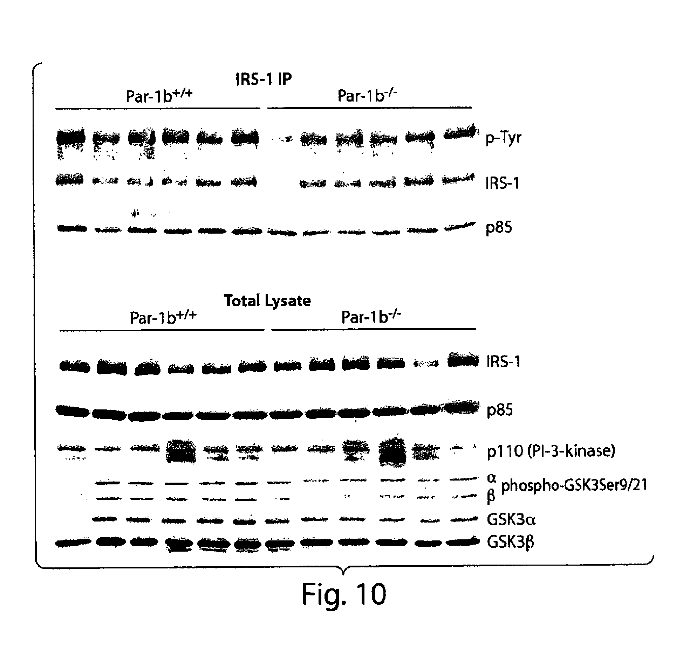

**Figure 10****. Insulin-stimulated BAT analysis indicates unchanged proximal insulin signals in Par-b^{-/-} mice.**
   Wild-type and Par-1b^{-/-} mice were injected (intraperitoneal) with a submaximal (0.3U/kg body weight) does of insulin. After 20' tissues were harvested and IRS-1 was immunoprecipitated from brown adipose tissue (top panel). Total cell lysates (50 µg) were also analyzed by immunoblotting with the indicated antibodies (bottom panel).

### Detailed Description of the Invention

Currently available type 2 diabetes therapeutics have not been molecularly or biochemically targeted in their development and the reason for their therapeutic success, however limited, is not known. Because of this, direct inhibitors of Par-1b signaling are expected to be more potent in their therapeutic potential. By directly inhibiting Par-1b signaling, one might achieve the highest possible restoration of insulin signaling in diabetic tissue, resulting in more potent, long lasting therapeutics. Based on this, we suggest screening for direct chemical inhibitors of Par-1b to identify potential therapeutic compounds. Such compounds are useful in the treatment of type 2 diabetes or any clinical conditions resulting from a loss of insulin responsiveness.

Determination of the catalytic activity of Par-1b polypeptide for diagnostic, prognostic, and therapeutic purposes is an aspect of the invention. The catalytic activity of a Par-1b polypeptide may be determined and candidate pharmaceutical agents can be tested for their ability to decrease the Par-1b catalytic activity, e.g., kinase activity. The determination that a compound decreases the Par-1b catalytic activity indicates that the compound may be useful as an agent to treat disorders involving a loss of insulin responsiveness, such as type 2 diabetes, or obesity. For example, a Par-1b polypeptide may be contacted with a substrate of the polypeptide and the kinase activity of the Par- 1b monitored and determined, then the Par-1b polypeptide may be contacted with a candidate agent and the polypeptide's kinase activity determined upon contact with the substrate. Such assays may be cell-based assays or *in vitro* assays and may also be useful to monitor effects of *in vivo* administration of inhibitors of Par-1b catalytic activity in cells or animals, including humans. *In vitro* assays can be, for example, polypeptide-based assays.

Par-1b is also known as MARK2 (MAP/microtubule affinity-regulating kinase 2); EMK1; and MGC99619. There are several splice variants of Par-1b known in the art and each of these can be utilized in the methods in order to identify compounds and compositions that inhibit Par-1b activity generally, or inhibit only the activity of one or more particular Par-1b splice variants. The sequence accession number for the long variant (transcript variant 1) of the human Par-1b gene is NM_017490.

The invention also relates in part to assays used to determine the activity of a Par-1b polypeptide, e.g., for screening inhibitors of these polypeptides. The Par-1b polypeptide may be attached to a surface and then contacted with a substrate molecule and the level of catalytic activity of the Par-1b polypeptide or fragment thereof can be monitored and quantitated using standard methods. The aforementioned assays are not intended to be limiting. Assays for catalytic activity may also be done with the components in solution, using various art-recognized detection methods, and/or other kinase assay methods known to one of ordinary skill in the art, some of which are described herein below. Typically these will be kinase assays as are well known in the art; certain examplary methods are provided in the Examples below.

The invention further provides efficient methods of identifying pharmacological agents or lead compounds for agents useful for decreasing Par-1b activity. Generally, the screening methods involve assaying for compounds that decrease (i.e., inhibit) phosphorylation of a substrate. Such methods are adaptable to automated, high throughput screening of compounds.

A wide variety of assays for pharmacological agents are provided, including labeled *in vitro* kinase phosphorylation assays, cell-based phosphorylation assays, assays for determining affinity of interacting proteins (e.g., immunoprecipitations, two-hybrid assays) etc. For example, in vitro kinase phosphorylation assays are used to rapidly examine the effect of candidate pharmacological agents on the phosphorylation of a substrate by, for example, Par-1b or a fragment thereof.

The candidate pharmacological agents can be derived from, for example, combinatorial peptide libraries, small molecule libraries, or natural product libraries.

In general, substrates used in the assay methods of the invention are added to an assay mixture as an isolated molecule. Substrates for Par-1b include tau, Dvl, Par-3, Cdc25C, MAP2 or MAP4 proteins. Still other substrates for the Par-1b kinase will be known to one of ordinary skill in the art. The assay mixture can include detectable phosphate compounds (e.g. ³²P or ³³P), so that protein substrates phosphorylated by Par-1b are readily detectable. Alternatively, Par-1b activity on a substrate can be measured using other detectable means such as antibody capture of specific phosphorylated polypeptides, chromatographic means, and other standard methods in the art.

A typical assay mixture for measuring kinase activity includes a protein substrate of the Par-1b kinase, or a peptide having a phosphorylation site motif of the substrate, and a candidate pharmacological agent. Typically, a plurality of assay mixtures are run in parallel with different agent concentrations to obtain a different response to the various concentrations. Typically, one of these concentrations serves as a negative control, i.e., at zero concentration of agent or at a concentration of agent below the limits of assay detection.

Candidate agents encompass numerous chemical classes, although typically they are organic compounds. Preferably, the candidate pharmacological agents are small organic compounds, i.e., those having a molecular weight of more than 50 yet less than about 2500. Candidate agents comprise functional chemical groups necessary for structural interactions with polypeptides (e.g., kinase sites), and typically include at least an amine, carbonyl, hydroxyl or carboxyl group, preferably at least two of the functional chemical groups and more preferably at least three of the functional chemical groups. The candidate agents can comprise cyclic carbon or heterocyclic structure and/or aromatic or polyaromatic structures substituted with one or more of the above-identified functional groups. Candidate agents also can be biomolecules such as peptides, saccharides, fatty acids, sterols, isoprenoids, purines, pyrimidines, derivatives or structural analogs of the above, or combinations thereof and the like. Where the agent is a nucleic acid (i.e., aptamer), the agent typically is a DNA or RNA molecule, although modified nucleic acids having non-natural bonds or subunits are also contemplated.

Par-1b inhibitors also can be designed using rational structure-based methods such as the methods described in PCT/US98/10876 and references described therein.

Candidate agents are obtained from a wide variety of sources including libraries of synthetic or natural compounds. For example, numerous methods are available and knowns to one of ordinary skill in the art for random and directed synthesis of a wide variety of organic compounds and biomolecules, including expression of randomized oligonucleotides, random or non-random peptide libraries, synthetic organic combinatorial libraries, phage display libraries of random peptides, and the like. Alternatively, libraries of natural compounds in the form of bacterial, fungal, plant and animal extracts are available or readily produced. Additionally, natural and synthetically produced libraries and compounds can be readily be modified through conventional chemical, physical, and biochemical means. Further, known pharmacological agents may be subjected to directed or random chemical modifications such as acylation, alkylation, esterification, amidification, etc. to produce structural analogs of the agents.

A variety of other reagents also can be included in the mixture. These include reagents such as salts, buffers, neutral proteins (e.g., albumin), detergents, etc. which may be used to facilitate optimal protein activity. Such a reagent may also reduce non-specific or background interactions of the reaction components. Other reagents that improve the efficiency of the assay such as nuclease inhibitors, antimicrobial agents, and the like may also be used.

The mixture of the foregoing assay materials is incubated under conditions whereby, but for the presence of the candidate pharmacological agent, Par-1b phosphorylates a polypeptide at a certain level (i.e., a control level). The order of addition of components, incubation temperature, time of incubation, and other parameters of the assay may be readily determined. Such experimentation merely involves optimization of the assay parameters, not the fundamental composition of the assay. Incubation temperatures typically are between 4°C and 40°C. Incubation times preferably are minimized to facilitate rapid, high throughput screening, and typically are between 1 second and 1 hour.

After incubation, the presence or absence of phosphorylation of a substrate, or binding of a substrate, is detected by any convenient method available to the user. For cell free assays, a separation step may be used to separate bound from unbound components. The separation step may be accomplished in a variety of ways. Conveniently, at least one of the components is immobilized on a solid substrate, from which the unbound components may be easily separated. The solid substrate can be made of a wide variety of materials and in a wide variety of shapes, e.g., microtiter plate, microbead, dipstick, resin particle, etc. The substrate preferably is chosen to maximum signal to noise ratios, primarily to minimize background binding, as well as for ease of separation and cost.

Separation may be effected for example, by removing a bead or dipstick from a reservoir, emptying or diluting a reservoir such as a microtiter plate well, rinsing a bead, particle, chromatographic column or filter with a wash solution or solvent. The separation step preferably includes multiple rinses or washes. For example, when the solid substrate is a microtiter plate, the wells may be washed several times with a washing solution, which typically includes those components of the incubation mixture that do not participate in specific binding or interaction such as salts, buffer, detergent, non-specific protein, etc. Where the solid substrate is a magnetic bead, the beads may be washed one or more times with a washing solution and isolated using a magnet.

Detection may be effected using any convenient method. For example, phosphorylation produces a directly or indirectly detectable product, e.g., phosphorylated substrate. In other assays, one of the components usually comprises, or is coupled to, a detectable label. A wide variety of labels can be used, such as those that provide direct detection (e.g., radioactivity, luminescence, fluorescence, optical or electron density, etc). or indirect detection (e.g., epitope tag such as the FLAG, V5 or *myc* epitopes, an enzyme tag such as horseradish peroxidase or luciferase, a transcription product, etc.). The label may be bound to a substrate, to the proteins employed in the assays, or to the candidate pharmacological agent. Phosphorylated substrates also can be assayed using IMAP technology (Molecular Devices, Sunnyvale, CA), that uses the specific binding of metal (MIII) coordination complexes to phosphate groups at high salt concentration and a fluorescence polarization readout.

A variety of methods may be used to detect the label, depending on the nature of the label and other assay components. For example, the label may be detected while bound to the solid substrate or subsequent to separation from the solid substrate. Labels may be directly detected through optical or electron density, radioactive emissions, nonradiative energy transfers, etc. or indirectly detected with antibody conjugates, streptavidin-biotin conjugates, etc. Methods for detecting the labels are well known in the art.

Thus the present invention includes automated drug screening assays for identifying compositions having the ability to increase phosphorylation of a substrate directly or indirectly. The automated methods preferably are carried out in an apparatus which is capable of delivering a reagent solution to a plurality of predetermined compartments of a vessel and measuring the change in a detectable molecule in the predetermined compartments. Exemplary methods include the following steps. First, a divided vessel is provided that has one or more compartments which contain a substrate which, when exposed to Par-1b, has a detectable change. The Par-1b can be in a cell in the compartment, in solution, or immobilized within the compartment. Next, one or more predetermined compartments are aligned with a predetermined position (e.g., aligned with a fluid outlet of an automatic pipette) and an aliquot of a solution containing a compound or mixture of compounds being tested for its ability to decrease Par-1b kinase activity is delivered to the predetermined compartment(s) with an automatic pipette. The substrate also can be added with the compounds or following the addition of the compounds. Finally, detectable signal; emitted by the substrate is measured for a predetermined amount of time, preferably by aligning the cell-containing compartment with a detector. Preferably, the signal also measured prior to adding the compounds to the compartments, to establish e.g., background and/or baseline (control) values. For competition assays, the compounds can be added with or after addition of a substrate or inhibitor to the Par-1b polypeptide-containing compartments. One of ordinary skill in the art can readily determine the appropriate order of addition of the assay components for particular assays.

At a suitable time after addition of the reaction components, the plate is moved, if necessary, so that assay wells are positioned for measurement of signal. Because a change in the signal may begin within the first few seconds after addition of test compounds, it is desirable to align the assay well with the signal detector as quickly as possible, with times of about two seconds or less being desirable. In preferred embodiments of the invention, where the apparatus is configured for detection through the bottom of the well(s) and compounds are added from above the well(s), readings may be taken substantially continuously, since the plate does not need to be moved for addition of reagent. The well and detector device should remain aligned for a predetermined period of time suitable to measure and record the change in signal.

The apparatus of the present invention is programmable to begin the steps of an assay sequence in a predetermined first well (or rows or columns of wells) and proceed sequentially down the columns and across the rows of the plate in a predetermined route through well number n. It is preferred that the data from replicate wells treated with the same compound are collected and recorded (e.g., stored in the memory of a computer) for calculation of signal.

To accomplish rapid compound addition and rapid reading of the response, the detector can be modified by fitting an automatic pipetter and developing a software program to accomplish precise computer control over both the detector and the automatic pipetter. By integrating the combination of the fluorometer and the automatic pipetter and using a microcomputer to control the commands to the detector and automatic pipetter, the delay time between reagent addition and detector reading can be significantly reduced. Moreover, both greater reproducibility and higher signal-to-noise ratios can be achieved as compared to manual addition of reagent because the computer repeats the process precisely time after time. Moreover, this arrangement permits a plurality of assays to be conducted concurrently without operator intervention. Thus, with automatic delivery of reagent followed by multiple signal measurements, reliability of the assays as well as the number of assays that can be performed per day are advantageously increased.

Similar assays can be used to identify compounds that increase Par-1b activity, which may be useful as controls or in preparing animal models of disease.

Inhibitors of Par-1b polypeptide activity (e.g., kinase activity) identified by the methods described herein are useful to treat diseases or conditions that result from increased or excessive Par-1b polypeptide activity, including disorders characterized by loss of (or reduced) insulin responsiveness (e.g., type 2 diabetes) and obesity. For treatment of such conditions, an effective amount of a Par-1b polypeptide inhibitor is administered to a subject.

As used herein a "control" may be a predetermined value, which can take a variety of forms. It can be a single cut-off value, such as a median or mean. It can be established based upon comparative groups, such as in groups having normal amounts of circulating insulin and groups having abnormal amounts of circulating insulin. Another example of comparative groups would be groups having a particular disease, condition or symptoms and groups without the disease, condition or symptoms, such as individuals diagnosed with type 2 diabetes mellitus and individuals free of type 2 diabetes mellitus. Another comparative group would be a group with a family history of a condition and a group without such a family history. The predetermined value can be arranged, for example, where a tested population is divided equally (or unequally) into groups, such as a low-risk group, a medium-risk group and a high-risk group or into quandrants or quintiles.

The predetermined value, of course, will depend upon the particular population selected. For example, an apparently healthy population will have a different 'normal' range than will a population which is known to have a condition related to increased Par-1b activity, or a disorder characterized by a loss of insulin responsiveness. Accordingly, the predetermined value selected may take into account the category in which an individual falls. Appropriate ranges and categories can be selected with no more than routine experimentation by those of ordinary skill in the art. By abnormal is meant statistically significantly different relative to a selected control. Typically the control will be based on apparently healthy normal individuals in an appropriate age bracket.

In some embodiments, a control sample is from a cell, tissue, or subject that does not have a disorder associated with Par-1b activity, a disorder characterized by a loss of insulin responsiveness, or obesity. In other embodiments the control sample is a sample that is untreated with a candidate agent. For example, an effect of a candidate agent may be determined by determining the catalytic activity of a Par-1b polypeptide (e.g., kinase activity) in advance of contacting the Par-1b polypeptide with the agent, and again after contacting the Par-1b polypeptide with the agent, in which case, the initial level of catalytic activity or binding determined may serve as a control level against which the post-contact level of catalytic or binding activity may be compared. In such assays, the source of the Par-1b polypeptide may be a biological sample from a subject known to be free of a disorder characterized by a loss of insulin responsiveness (e.g., type 2 diabetes mellitus) or may be a sample from a cell or tissue from a subject with a known disorder characterized by a loss of insulin responsiveness, and in each case the before-contact determination of catalytic activity may be the control for the after-contact determination of catalytic activity.

Examples of methods for obtaining the biological sample from the tissue biopsy of a subject include aspiration, gross apportioning of a mass, microdissection, laser-based microdissection, or other art-known cell-separation methods. Fluid biological samples are taken from a subject using standard methods well known in the art.

Because of the variability of the cell types in tissue biopsy material, and the variability in sensitivity of the diagnostic methods used, the sample size required for analysis may range from 1, 10, 50, 100, 200, 300, 500, 1000, 5000, 10,000, to 50,000 or more cells. The appropriate sample size may be determined based on the cellular composition and condition of the biopsy and the standard preparative steps for this determination and subsequent isolation of fractions of the sample (e.g., polypeptides, nucleic acids) for use in the invention are well known to one of ordinary skill in the art. An example of this, although not intended to be limiting, is that in some instances a sample from the biopsy may be sufficient for assessment of RNA expression without amplification, but in other instances the lack of suitable cells in a small biopsy region may require use of RNA conversion and/or amplification methods or other methods to enhance resolution of the nucleic acid molecules. Such methods, which allow use of limited biopsy materials, are well known to those of ordinary skill in the art and include, but are not limited to: direct RNA amplification, reverse transcription of RNA to cDNA, real-time RT-PCR, amplification of cDNA, or the generation of radiolabeled nucleic acids.

Any method for decreasing Par-1b activity will be useful in the treatment of the aforementioned disorders characterized by loss of insulin responsiveness (e.g., type 2 diabetes). Par-1b activity can be decreased by pharmacological inhibitors of the enzyme activity or the expression of the proteins. Preferred inhibitors are those that bind directly to the Par-1b proteins.

Agents that decrease the expression of Par-1b (e.g., by decreasing expression of the endogenous gene) also can be used for these purposes. Such agents include antisense nucleic acid molecules and siRNA or RNAi molecules. In accordance with the invention, known inhibitors of Par-1b that can be used include the siRNA sequence AAG ACU CAA CUG AAC UCC UCC (SEQ ID NO:1) corresponding to coding nucleotides 256-276 of Par-1b (see reference 22), or the sequence used in Tang et al. PNAS. 2006, 103:2087-92.

One set of embodiments of the aforementioned compositions and methods include the use of antisense molecules or nucleic acid molecules that reduce expression of genes via RNA interference (RNAi or siRNA). One example of the use of antisense, RNAi or siRNA in the methods of the invention is their use to decrease the level of expression of one or more ceramide biosynthetic pathway enzymes. The antisense oligonucleotides, RNAi, or siRNA nucleic acid molecules used for this purpose may be composed of "natural" deoxyribonucleotides, ribonucleotides, or any combination thereof. That is, the 5' end of one native nucleotide and the 3' end of another native nucleotide may be covalently linked, as in natural systems, via a phosphodiester internucleoside linkage. These oligonucleotides may be prepared by art-recognized methods, which may be carried out manually or by an automated synthesizer. They also may be produced recombinantly by vectors.

In some embodiments of the invention, the antisense or siRNA oligonucleotides also may include "modified" oligonucleotides. That is, the oligonucleotides may be modified in a number of ways, which do not prevent them from hybridizing to their target but which enhance their stability or targeting or which otherwise enhance their therapeutic effectiveness.

The term "modified oligonucleotide" as used herein describes an oligonucleotide in which (1) at least two of its nucleotides are covalently linked via a synthetic internucleoside linkage (i.e., a linkage other than a phosphodiester linkage between the 5' end of one nucleotide and the 3' end of another nucleotide) and/or (2) a chemical group not normally associated with nucleic acids has been covalently attached to the oligonucleotide. Preferred synthetic internucleoside linkages are phosphorothioates, alkylphosphonates, phosphorodithioates, phosphate esters, alkylphosphonothioates, phosphoramidates, carbamates, carbonates, phosphate triesters, acetamidates, carboxymethyl esters and peptides.

The term "modified oligonucleotide" also encompasses oligonucleotides with a covalently modified base and/or sugar. For example, modified oligonucleotides include oligonucleotides having backbone sugars that are covalently attached to low molecular weight organic groups other than a hydroxyl group at the 3' position and other than a phosphate group at the 5' position. Thus, modified oligonucleotides may include a 2'-O-alkylated ribose group. In addition, modified oligonucleotides may include sugars such as arabinose instead of ribose. The present invention, thus, contemplates pharmaceutical preparations containing modified antisense molecules that are complementary to and hybridizable with, under physiological conditions, nucleic acid molecules encoding proteins of the invention, together with pharmaceutically acceptable carriers.

The use of RNA interference or "RNAi" involves the use of double-stranded RNA (dsRNA) to block gene expression. (see: Sui, G, et al, Proc Natl. Acad. Sci U.S.A. 99:5515-5520,2002). Methods of applying RNAi strategies in embodiments of the invention would be understood by one of ordinary skill in the art.

Methods in which small interfering RNA (siRNA) molecules are used to reduce the expression of Par-1b may be used. In one aspect, a cell is contacted with a siRNA molecule to produce RNA interference (RNAi) that reduces expression of one or more of the aforementioned genes. The siRNA molecule is directed against nucleic acids coding for the relevant polypeptide (e.g. RNA transcripts including untranslated and translated regions). Well known methods such as Western blotting can be used for determining the level of protein expression and Northern blotting or RT-PCR can be used for determining the level of mRNA transcript of the targeted gene.

As used herein, a "siRNA molecule" is a double stranded RNA molecule (dsRNA) consisting of a sense and an antisense strand. The antisense strand of the siRNA molecule is a complement of the sense strand (Tuschl, T. et al., 1999, Genes & Dev., 13:3191-3197; Elbashir, S.M. et al., 2001, EMBO J., 20:6877-6888; incorporated herein by reference). In one embodiment the last nucleotide at the 3' end of the antisense strand may be any nucleotide and is not required to be complementary to the region of the target gene. The siRNA molecule preferably is 19-23 nucleotides in length and may form a hairpin structure. In one preferred embodiment the siRNA molecule includes a two nucleotide 3' overhang on the sense strand. In a second preferred embodiment the two nucleotide overhang is thymidine-thymidine (TT). The siRNA molecule corresponds to at least a portion of a target gene. In one embodiment the siRNA molecule corresponds to a region selected from a cDNA target gene beginning between 50 to 100 nucleotides downstream of the start codon. In a preferred embodiment the first nucleotide of the siRNA molecule is a purine.

The siRNA molecules can be plasmid-based. In a preferred method, a polypeptide encoding sequence of one of the aforementioned genes is amplified using the well known technique of polymerase chain reaction (PCR). The use of the entire polypeptide encoding sequence is not necessary; as is well known in the art, a portion of the polypeptide encoding sequence is sufficient for RNA interference. The PCR fragment is inserted into a vector using routine techniques well known to those of skill in the art. Combinations of the foregoing can be expressed from a single vector or from multiple vectors introduced into cells.

In one aspect of the invention, a mammalian vector comprising any of the nucleotide coding sequences of the invention is provided. The mammalian vectors include but are not limited to the pSUPER RNAi vectors (Brummelkamp, T.R. et al., 2002, Science, 296:550-553, incorporated herein by reference). In one embodiment, a nucleotide coding sequence can be inserted into the mammalian vector using restriction sites, creating a stem-loop structure. In a second embodiment, the mammalian vector may comprise the polymerase-III H1-RNA gene promoter. The polymerase-III H1-RNA promoter produces a RNA transcript lacking a polyadenosine tail and has a well-defined start of transcription and a termination signal consisting of five thymidines (T5) in a row. The cleavage of the transcript at the termination site occurs after the second uridine and yields a transcript resembling the ends of synthetic siRNAs containing two 3' overhanging T or U nucleotides. The antisense strand of the siRNA molecule hybridizes to the corresponding region of the mRNA of the target gene.

Preferred systems for mRNA expression in mammalian cells are those such as pSUPER RNAi system as described in Brummelkamp et al. (2002, Science, 296:550-553). Other examples include but are not limited to pSUPER.neo, pSUPER.neo+gfp, pSUPER.puro, BLOCK-iT T7-TOPO linker, pcDNA1.2/V5-GW/lacZ, pENTR/U6, pLenti6-GW/L16-laminshrna, and pLenti6/BLOCK-iT-DEST. These vectors are available from suppliers such as Invitrogen, and one of skill in the art would be able to obtain and use them.

Treatment for a disorder resulting from loss of insulin responsiveness may include, but is not limited to, dietetic therapy and pharmaceutical therapy. In some embodiments, treatment may include administration of a pharmaceutical agent that decreases Par-1b activity. The inhibitors of Par-1b activity can be administered in conjunction with other pharmaceutical agents known for treatment of such disorders. For example, in the treatment of type 2 diabetes, other therapeutics such as insulin sensitizers, insulin secretagogues, insulin, and the like, can be administered in conjunction (simultaneously or sequentially) with therapeutics that decrease Par-1b activity or expression. For treatment of obesity, bariatric surgery can be used in conjunction with the compounds and compositions of the invention. Likewise, other obsity therapeutics such as Sibutramine (Meridia; Abbott Laboratories) and Orlistat (Xenical; Roche), and the like, can be administered in conjunction (simultaneously or sequentially) with therapeutics that decrease Par-1b activity or expression.

As used herein, a subject is preferably a human, non-human primate, cow, horse, pig, sheep, goat, dog, cat, or rodent. In all embodiments, human subjects are preferred. In some embodiments, the subject is suspected of having a disorder associated with abnormally increased Par-1b activity (relative to healthy individuals), which results in or results from a loss of insulin responsiveness, such as type II diabetes.

Methods for identifying subjects having or suspected of having a disorder associated with abnormally increased Par-1b activity, a disorder characterized by a loss of insulin responsiveness, or obesity include, but are not limited to: physical examination, analysis of a subject's medical history, analysis of a subject's family medical history, blood tests, visual exam, mean body mass assessment, and/or weight assessment. Diagnostic methods for identifying subjects having or suspected of having a disorder characterized by a loss of insulin responsiveness are well-known to those of skill in the medical arts, although not necessarily with respect to increased Par-1b activity.

As used herein, a biological sample includes, but is not limited to: tissue, cells, or body fluid (e.g., blood or lymph node fluid). The fluid sample may include cells and/or fluid. The tissue and cells may be obtained from a subject or may be grown in culture (e.g., from a cell line). In some embodiments of the invention, the biological sample is a control sample.

The amino acid sequences identified herein as Par-1b polypeptides, and the nucleotide sequences encoding them, are sequences deposited in databases such as GenBank as known to those skilled in the art. The use of these known Par-1b sequences in pharmaceutical screening assays, determination of pharmaceutical agents, and diagnostic assays for disorders resulting from loss of insulin responsiveness, e.g., type 2 diabetes, as described herein is novel. Homologs, alleles, orthologs and other variants of the Par-1b nucleic acid sequences and polypeptides sequences can also be used, as appropriate, as will be known to one of ordinary skill in the art. In general, homologs, alleles and other variants typically will share at least 90% nucleotide identity and/or at least 95% amino acid identity to the sequences of a Par-1b nucleic acid and polypeptide, respectively, in some instances will share at least 95% nucleotide identity and/or at least 97% amino acid identity, and in other instances will share at least 97% nucleotide identity and/or at least 99% amino acid identity. The homology can be calculated using various, publicly available software tools developed by NCBI (Bethesda, Maryland) that can be obtained through the Internet or using a variety of commercially available softward packages. Exemplary tools include the BLAST system available from the website of the National Center for Biotechnology Information (NCBI) at the National Institutes of Health.

The identification herein of Par-1b polypeptides as involved in physiological disorders involving a loss of insulin responsiveness also permits the artisan to diagnose a disorder characterized by expression of Par-1b polypeptides, and characterized preferably by an alteration in functional activity of the Par-1b polypeptides.

Thus the invention also includes methods to monitor the onset, progression, or regression of a disorder associated with increased or excessive Par-1b activity in a subject by, for example, obtaining samples at sequential times from a subject and assaying such samples for the level of expression of Par-1b nucleic acid molecules, the level of expression of Par-1b polypeptide molecules, and/or the level of activity of a Par-1b polypeptide (e.g., kinase activity). A subject may be suspected of having a disorder associated with increased or excessive Par-1b activity or may be believed not to have such a disorder and in the latter case, the sample expression or activity level may serve as a control for comparison with subsequent samples.

Onset of a condition is the initiation of the changes associated with the condition in a subject. Such changes may be evidenced by physiological symptoms, or may be clinically asymptomatic. For example, the onset of a disorder associated with increased or excessive Par-1b activity may be followed by a period during which there may be physiological changes in the subject, even though clinical symptoms may not be evident at that time. The progression of a condition follows onset and is the advancement of the physiological elements of the condition, which may or may not be marked by an increase in clinical symptoms. Onset and progression are similar in that both represent an increase in the characteristics of a disorder (e.g., expression or activity of Par-1b molecules), in a cell or subject, onset represents the beginning of this disorder and progression represents the worsening of a preexisting condition. In contrast to onset and progression, regression of a condition is a decrease in physiological characteristics of the condition, perhaps with a parallel reduction in symptoms, and may result from a treatment or may be a natural reversal in the condition.

A marker for disorders associated with increased or excessive Par-1b activity may be the level or amount of catalytic activity of a Par-1b polypeptide, the level or amount of specific phosphorylation of a Par-1b substrate, or the level of expression of a Par-1b nucleic acid or polypeptide.

The invention also involves the use of agents such as polypeptides that bind to Par-1b polypeptides or substrates of such polypeptides. Such binding agents can be used, for example, in screening assays to detect the presence or absence of Par-1b polypeptides or their substrates and in purification protocols to isolate Par-1b, their substrates or complexes of Par-1b polypeptides and their substrates.

The invention, therefore, embraces peptide binding agents which, for example, can be antibodies or fragments of antibodies having the ability to selectively bind to Par-1b polypeptides or their substrates. Antibodies include polyclonal and monoclonal antibodies, prepared according to conventional methodology. As used herein, Par-1b antibodies, are antibodies that specifically bind to Par-1b polypeptides.

Significantly, as is well known in the art, only a small portion of an antibody molecule, the paratope, is involved in the binding of the antibody to its epitope (see, in general, Clark, W.R. (1986) The Experimental Foundations of Modern Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDR3). The CDRs, and in particular the CDR3 regions, and more particularly the heavy chain CDR3, are largely responsible for antibody specificity.

It is now well established in the art that the non-CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody. See, e.g., U.S. patents 4,816,567, 5,225,539, 5,585,089, 5,693,762 and 5,859,205.

Fully human monoclonal antibodies also can be prepared by immunizing mice transgenic for large portions of human immunoglobulin heavy and light chain loci. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (HAMA) responses when administered to humans.

Thus, as will be apparent to one of ordinary skill in the art, the present invention also provides for F(ab')₂, Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')₂ fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present invention also includes so-called single chain antibodies.

Thus, the invention involves polypeptides of numerous size and type that bind specifically to Par-1b polypeptides, their substrates and complexes of both Par-1b polypeptides and their substrates. These polypeptides may be derived also from sources other than antibody technology. For example, such polypeptide binding agents can be provided by degenerate peptide libraries which can be readily prepared in solution, in immobilized form or as phage display libraries. Combinatorial libraries also can be synthesized of peptides containing one or more amino acids. Libraries further can be synthesized of peptoids and non-peptide synthetic moieties.

Phage display can be particularly effective in identifying binding peptides useful according to the invention. Briefly, one prepares a phage library (using e.g. m13, fd, or lambda phage), displaying inserts from 4 to about 80 amino acid residues using conventional procedures. The inserts may represent, for example, a completely degenerate or biased array. One then can select phage-bearing inserts which bind to the Par-1b polypeptide. This process can be repeated through several cycles of reselection of phage that bind to the Par-1b polypeptide. Repeated rounds lead to enrichment of phage bearing particular sequences. DNA sequence analysis can be conducted to identify the sequences of the expressed polypeptides. The minimal linear portion of the sequence that binds to the Par-1b polypeptide can be determined. One can repeat the procedure using a biased library containing inserts containing part or all of the minimal linear portion plus one or more additional degenerate residues upstream or downstream thereof. Yeast two-hybrid screening methods also may be used to identify polypeptides that bind to the Par-1b polypeptides.

The invention also relates in part to methods of treating disorders associated with abnormally increased Par-1b activity, which include a loss of insulin responsiveness, such as type 2 diabetes, and obesity. An "effective amount" of a drug therapy is an amount of an agent that decreases Par-1b activity that alone, or together with further doses, produces the desired response, e.g. reduction of symptoms of type 2 diabetes, or other disease associated with a loss of insulin responsiveness, or obesity.

In the case of treating a particular disease or condition the desired response is inhibiting the progression of the disease or condition. This may involve only slowing the progression of the disease temporarily, although more preferably, it involves halting the progression of the disease permanently. This can be monitored by routine diagnostic methods known to one of ordinary skill in the art for any particular disease. The desired response to treatment of the disease or condition also can be delaying the onset or even preventing the onset of the disease or condition, or reversing the physiological effects of the disease.

Such amounts will depend, of course, on the particular condition being treated, the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the agent that decreases Par-1b activity (alone or in combination with other therapeutic agents) be used, that is, the highest safe dose according to sound medical judgment. It will be understood by those of ordinary skill in the art, however, that a patient may insist upon a lower dose or tolerable dose for medical reasons, psychological reasons or for virtually any other reasons.

The pharmaceutical compositions used in the foregoing methods preferably are sterile and contain an effective amount of an agent that decreases Par-1b activity for producing the desired response in a unit of weight or volume suitable for administration to a patient.

The doses of an agent that decreases Par-1b activity administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

Various modes of administration will be known to one of ordinary skill in the art which effectively deliver the agent that decreases Par-1b activity to a desired tissue, cell or bodily fluid. Administration includes: topical, intravenous, oral, intracavity, intrathecal, intrasynovial, buccal, sublingual, intranasal, transdermal, intravitreal, subcutaneous, intramuscular and intradermal administration. The invention is not limited by the particular modes of administration disclosed herein. Standard references in the art (e.g., Remington's Pharmaceutical Sciences, 18th edition, 1990) provide modes of administration and formulations for delivery of various pharmaceutical preparations and formulations in pharmaceutical carriers. Other protocols which are useful for the administration of agent that decreases Par-1b activity will be known to one of ordinary skill in the art, in which the dose amount, schedule of administration, sites of administration, mode of administration (e.g., intra-organ) and the like vary from those presented herein.

Administration to mammals other than humans of agents that decrease Par-1b activity, e.g. for testing purposes or veterinary therapeutic purposes, is carried out under substantially the same conditions as described above. It will be understood by one of ordinary skill in the art that this invention is applicable to both human and animal diseases that can be treated by an agent that decreases Par-1b activity. Thus this invention is intended to be used in husbandry and veterinary medicine as well as in human therapeutics.

In general, a therapeutically effective amount of an agent that decreases Par-1b activity typically varies from about 0.01 ng/kg to about 1000 µg/kg, preferably from about 0.1 ng/kg to about 200 µg/kg and most preferably from about 0.2 ng/kg to about 20 µg/kg, in one or more dose administrations daily, for one or more days. Lesser or greater amounts may be found to be therapeutically effective and thus also are useful in accordance with the invention. For treatments of disorders, it is preferred that doses of agents that decrease Par-1b activity are formulated and administered in doses between 0.2mg to 5000mg of the agent that decreases Par-1b activity. More preferably, an effective amount will be in the range from about 0.5mg to 500mg of the agent that increases Par-1b activity, according to any standard procedure in the art. Administration of agents that decrease Par-1b activity compositions to mammals other than humans, e.g. for testing purposes or veterinary therapeutic purposes, is carried out under substantially the same conditions as described above.

The pharmaceutical preparations of the invention may be administered alone or in conjunction with standard treatment(s) of disorders associated with alterations in Par-1b activity, such as type 2 diabetes or other disorders associated with a loss (i.e., a reduction or complete loss) of insulin responsiveness, and obesity. For example, treatment for type 2 diabetes with a pharmaceutical agent of the invention, may be undertaken in parallel with treatments for diabetes that is known and practiced in the art. For example, such treatments may include, but are not limited to administration of metformin, pioglitazone, and/or rosiglitazone. Other known treatments for type 2 diabetes include pharmaceutical agents that increases insulin release, which may include, but are not limited to sulfonylureas, nateglinide and repaglinide. In some treatment methods, sulfonylureas include, but are not limited to glibenclamide (glyburide), gliclazide and glimepiride. In some embodiments of the invention, insulin may be administered to the subject, in conjunction with the treatment methods of the invention.

When administered, the pharmaceutical preparations of the invention are applied in pharmaceutically-acceptable amounts and in pharmaceutically-acceptable compositions. The term "pharmaceutically acceptable" means a non-toxic material that does not interfere with the effectiveness of the biological activity of the active ingredients. Such preparations may routinely contain salts, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts. Preferred components of the composition are described above in conjunction with the description of the agent that decreases Par-1b activity of the invention.

An agent that decreases Par-1b activity may be combined, if desired, with a pharmaceutically-acceptable carrier. The term "pharmaceutically-acceptable carrier" as used herein means one or more compatible solid or liquid fillers, diluents or encapsulating substances which are suitable for administration into a human. The term "carrier" denotes an organic or inorganic ingredient, natural or synthetic, with which the active ingredient is combined to facilitate the application. The components of the pharmaceutical compositions also are capable of being co-mingled with the agent that decreases Par-1b activity, and with each other, in a manner such that there is no interaction which would substantially impair the desired pharmaceutical efficacy.

The pharmaceutical compositions may contain suitable buffering agents, as described above, including: acetate, phosphate, citrate, glycine, borate, carbonate, bicarbonate, hydroxide (and other bases) and pharmaceutically acceptable salts of the foregoing compounds.

The pharmaceutical compositions also may contain, optionally, suitable preservatives, such as: benzalkonium chloride; chlorobutanol; parabens and thimerosal.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous liquids such as a syrup, elixir or an emulsion.

Compositions suitable for parenteral administration conveniently comprise an agent that decreases Par-1b activity. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Carrier formulation suitable for oral, subcutaneous, intravenous, intramuscular, etc. administrations can be found in Remington's Pharmaceutical Sciences, Mack Publishing Co., Easton, PA.

A long-term sustained release implant also may be used for administration of the pharmaceutical agent composition. "Long-term" release, as used herein, means that the implant is constructed and arranged to deliver therapeutic levels of the active ingredient for at least 30 days, and preferably 60 days. Long-term sustained release implants are well known to those of ordinary skill in the art and include some of the release systems described above. Such implants can be particularly useful in treating conditions characterized by Par-1b activity by placing the implant near portions of a subject affected by such activity, thereby effecting localized, high doses of the compounds of the invention.

The invention also includes kits for assaying the activity level of a Par-1b polypeptide (e.g., kinase activity) for determining whether test compounds decrease the Par-1b polypeptide activity. One example of such a kit of the invention is a kit that provides components necessary to determine the activity level of a Par-1b polypeptide of the invention using a kinase assay. The components can include an appropriate substrate molecule as well as necessary cofactors and other components (e.g., buffers, radioactive molecules).

Another example of a kit of the invention, is a kit that provides components necessary to determine the level of expression of a Par-1b nucleic acid molecule of the invention. Such components may include, primers useful for amplification of a Par-1b nucleic acid molecule and/or other chemicals for PCR amplification.

The foregoing kits can include instructions or other printed material on how to use the various components of the kits for diagnostic purposes or for compound screening purposes.

### Examples

### Introduction

The Par-1b/MARK2/Emk gene product is implicated in the regulation of a number of cellular processes, including the generation and/or maintenance of cell polarity. Originally identified in *Caenorhabdhitis elegans* as a critical player in early embryonic polarity, Par-1 has since been shown to regulate cell polarity in yeast, *Drosophila, Xenopus* and several mammalian cell types (1-13). Par-1 is a regulator of microtubule stability, the Wnt signaling pathway and also plays a role in vesicular trafficking (2, 8, 12, 14-19). Two critical regulators of Par-1, LKB1 (Par-4) and atypical PKCζ/λ, (aPKC), have been identified in multiple systems. LKB1 catalyzes phosphorylation of an activation loop Thr residue in the kinase domain of the Par-1, while aPKC phosphorylates a conserved Thr residue in the "spacer" domain of the Par-1 kinases (20-22). In contrast to the activating phosphorylation catalyzed by LKB1, aPKC negatively regulates localization and activity of Par-1(22-25).

In mammals, four Par-1 family members exist (Par-1b/MARK2/Emk, Par-1a/C-TAK1/MARK3, PAR-1c/MARK1, Par-1d/MARK4/MARKL1), each encoded by distinct genes and each expressed selectively in multiple tissues (26-29). Two studies have been published on the generation and characterization of mice null for one of these genes, Par-1b/MARK2/Emk (30, 31). Bessone et al. (30) characterized defects in growth and fertility, while we previously reported a role for Par-1b in immune system homeostasis (Hurov et al., 2001). Here, we demonstrate that Par-1b null mice display a number of striking metabolic disorders. These include reduced adiposity, hypoinsulinemia, insulin hypersensitivity, resistance to high-fat diet induced weight gain and increased glucose uptake into white and brown adipose tissue. These findings elucidate a novel role for Par-1b in the regulation of both glucose homeostasis and energy balance and suggest that this kinase may be a useful target for the treatment of Type 2 diabetes and obesity.

### Results

### Par-1b null mice have reduced body weight and decreased adiposity

Generation of Par-1b kinase null mice (Par-1b^{-/-}) has been described previously (31). Par-1b^{-/-} mice (males and females) weigh ∼20% less than their wild-type littermates during both embryonic (118 ±12 mg versus 150 ±16 mg, 13.5 days post coitus, p<0.005) and postnatal growth (Figure 1A-C). Par-1b heterozygotes (Par-1b^{+/-}) also display statistically significant reductions in body weight at 1 year of age. Nose to anus length at 1 year of age was significantly reduced (∼8%) in null males (9.35 cm ± 0.57 Par-b^{-/-} versus 10.10cm ± 0.42 Par-b^{+/+}, p=0.04) and females (9.08 ± 0.35 Par-1b^{-/-} versus 9.75 ± 0.32 Par-1b^{+/+}, p=0.01).

It has been reported that Par-1b^{-/-} mice have reduced serum levels of IGF-1 (30). Since both pre- and post-natal growth retardation can result from IGF-1 deficiencies, we assayed levels of IGF-1 at 7 weeks of age in Par-1b null and wild-type mice. Par-1b^{-/-} mice have decreased serum IGF-1 levels (∼25% reduction in Par-1b^{-/-} relative to wild-type) at 7 weeks of age (535.5 ng/ml Par-1b^{-/-} versus 682.0 ng/ml Par-1b^{+/+}, p=0.015). Postnatally, IGF-1 transcription is driven primarily by growth hormone. We therefore quantitated serum growth hormone levels at 7 weeks of age (n=10 mice per genotype) and found no significant differences in single timepoint measurements (93.5±117 ng/ml Par-1b^{-/-} versus 56.2±111 ng/ml Par-1b^{+/+}, p=0.464). This data must be considered with the caveat that growth hormone is released in a pulsatile manner and that the single timepoint measurement may not be a particularly accurate readout of daily average growth hormone levels.

As reported previously (30), measurements of organ weight in Par-1b^{-/-} mice indicated that decreased organ weights were consistent with decreases in body mass (data not shown). However, proton magnetic resonance spectroscopy (¹H-MRS) and dissection of adipose tissue showed that Par-1b null mice have disproportionate decreases in fat mass relative to wild-type mice (2.47 g in Par-1b^{-/-}, versus 6.85 g Par-1b^{+/+}, epididymal fat pads, p<0.0001) at 12 weeks of age (Figure 1D). Similarly, quantitation of brown adipose tissue from intrascapular depots indicates 0.17±0.05 g in Par-1b⁻¹⁻ mice versus 0.44±0.14 g in Par-1b^{+/+} mice, n=10 mice/genotype, p<0.0001. The decrease in fat mass corresponds to ∼14% body fat in Par-1b^{-/-} mice versus ∼23% body fat in wild-type littermates (p<0.0001) (Figure 1E). Histological analysis of adipose tissue indicates that adipocyte size (white and brown adipose tissue, WAT and BAT) was comparable in Par-1b null and wild-type mice (Figure 1F). Adipocyte counts from multiple independent samples of white adipose tissue indicated no differences in adipocyte numbers per field of view from Par-1b wild-type and null WAT (72.4 ±3.7 adipocytes Par-1b^{+/+}, versus 72.2 ± 2.2 Par-1b^{-/-}, n= 5 and 9, respectively, p=0.96). Thus, the observed decrease in adiposity of Par-1b^{-/-} mice is due to decreases in total adipocyte cell number, not cell size.

### Par-1b null mice are resistant to diet-induced obesity and are hypermetabolic.

Based on the finding that Par-1b null mice are disproportionately lean, we sought to determine if Par-1b^{-/-} mice are resistant to weight gain when fed a high fat diet. Par-1b null and wild-type mice were placed on a high fat diet (42% fat by caloric content) for 16 weeks and monitored for body weight changes and food intake. Par-1b^{-/-} male mice are resistant to weight gain relative to wild-type controls (21.37g Par-1b^{-/-} versus 38.25g Par-1b^{+/+} at 20 weeks, p=0.005, Figure 2A). Female Par-1b null mice do not exhibit resistance to high fat diet induced weight gain (data not shown), although female Par-1b^{-/-} mice, like Par-1b^{-/-} males, are lean on a regular chow diet (data not shown). Serum levels of insulin, leptin, adiponectin, free fatty acids, triglycerides, and cholesterol were measured in order to elucidate potential mechanisms by which Par-1b^{-/-} mice were resistant to high fat diet induced weight gain (Table 1).

**Table 1**

| Metabolic characteristics of Par-1 b wild-type (WT) and null (KO) animals fed a high fat diet for 16 weeks | | | | | | |
|---|---|---|---|---|---|---|
| | Insulin ng/ml | Leptin ng/ml | Adiponectin ug/ml | Free Fatty Acids umol/l | Triglycerides mg/dl | Cholesterol mg/dl |
| WT | 381±119 | 34 75±7 23 | 17 38±4 92 | 1550 20+160 11 | 117 80±15 70 | 206 6+17 16 |
| KO | 0 75±0 09 | 6 94+1 21 | 12 89+2 44 | 1179 25+184 05 | 76 50+3 78 | 141 00+10 76 |
| P | 0057 | 0 012* | 0477 | 0 171 | 0057 | 0 019* |

| | | | | | | |
|---|---|---|---|---|---|---|
| 20 week old male mice (n=12 per genotype) were fasted for 5 hours Serum values are expressed as the mean±SEM of measurements obtained P represents p-value of student t-test (*significant) | | | | | | |

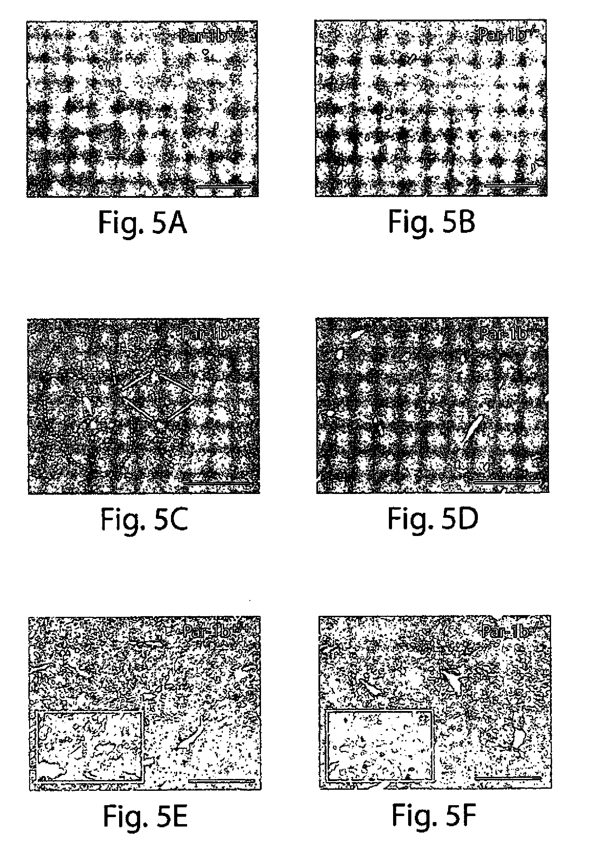

Leptin, an adipocyte-secreted hormone, is known to reduce feeding responses and increase insulin responsiveness and energy expenditure (32). Par-1b null mice exhibit 5-fold lower levels of leptin relative to wild-type mice after high fat diet feeding (6.94 ng/ml Par-1b^{-/-} versus 34.75 ng/ml Par-1b^{+/+}, p=0.012). This reduction in leptin levels is consistent with reduced adiposity in Par-1b null mice. A five-fold reduction in serum insulin levels (0.75 ng/ml Par-1b^{-/-} versus 3.80 ng/ml Par-1b^{+/+}, p=0.057) was observed, while triglyceride (76.50 mg/dl Par-1b^{-/-} versus 117.80 mg/dl Par-1b^{+/+}, p=0.057) and cholesterol (141.6 mg/dl Par-1b^{-/-} versus 206.6 mg/dl Par-1b^{+/+}, p=0.019) levels were also lower in Par-1b^{-/-} mice. Levels of adiponectin, unlike leptin, are not statistically different in Par-1b null mice, although there was a trend towards lower levels of adiponectin in Par-1b null mice (12.89 ug/ml Par-1b^{-/-} versus 17.38 ug/ml Par-1b^{+/+}, p=0.477). Histological analysis of aged mice (6 month old males, normal chow diet) also indicates that Par-1b^{-/-} mice are resistant to the onset of hepatic steatosis that was observed in wild-type littermates (Figure 5). This lack of hepatic fat accumulation in Par-1b^{-/-} mice is consistent with their resistance to high fat diet-induced obesity and reduction in white and brown fat depots.

Decreased adiposity and resistance to weight gain suggested that Par-1b^{-/-} mice might exhibit alterations in metabolic rate and energy expenditure. We measured total daily energy expenditure (EE), total metabolic rate (MR) and respiratory quotients (RQ) of male mice fed a high fat diet. Par-1b^{-/-} mice exhibit higher MR (7% increase), daily EE (27% increase), and food intake (2-fold increase) than wild-type littermates (Figure 2B-E). The respiratory quotient (RQ= VCO2/V02) of Par-1b null mice is not different than that of wild-type mice, suggesting that although these mice are hypermetabolic, they use similar ratios of carbohydrate and fats as food energy (Figure 2E). Par-1b^{-/-} mice are not significantly more or less active than wild-type mice (89.5±30.0 Par-1b^{+/+} versus 83.5±20.7 Par-1b^{-/-} activity counts). Body temperatures of Par-1b^{-/-} mice are slightly higher than wild-type littermates, although this difference is not statistically significant (96.4°C Par-1b^{+/+} versus 96.9°C Par-1b^{/}, p=0.13, n=10 mice/genotype).

### Enhanced insulin sensitivity and glucose uptake in adipose tissue in Par-1b^{-/-} mice.

Since Par-1b null mice are lean, hypermetabolic and have reduced serum insulin levels when fed a high fat diet, we hypothesized that these mice might have enhanced sensitivity to insulin. Consistent with measurements made in mice fed a high fat diet (Table 1), serum insulin levels were significantly lower in Par-1b null mice relative to their wild-type littermates under both fed and fasting conditions on a standard chow diet (Figure 3A). In spite of being hypoinsulinemic, Par-1b^{-/-} mice were normo-glycemic under fasting conditions and mildly hypoglycemic under fed conditions (Figure 3B). Insulin tolerance tests revealed that Par-1b null mice were indeed more sensitive to insulin than their wild-type littermates (Figure 3C). Par-1b null mice also exhibited enhanced glucose tolerance as assessed by glucose tolerance tests (Figure 3D), indicating that pancreatic beta cell function was more than sufficient to cope with an exogenously supplied glucose bolus. To further analyze insulin sensitivity in Par-1b null mice, hyperinsulinemic-euglycemic clamp studies were performed as described (33). The glucose infusion rate necessary to maintain euglycemia in the presence of a constant infusion of insulin (2.5mU/kg/min) was 35% higher (*P* = 0.0284) in Par-1b null mice when compared with wild-type littermates (Table 2). Accordingly, the insulin-stimulated whole-body glucose turnover was increased in Par-1b null mice relative to WT mice (*P* = 0.0364). These experiments confirm ITT analyses (Figure 3C), indicating that Par-1b null mice exhibit enhanced peripheral insulin sensitivity. Basal and clamp hepatic glucose production were not significantly different in Par-1b null mice. Since insulin clamp resulted in a complete suppression of hepatic glucose production in both groups of mice, we cannot exclude the possible effects of a lower-dose insulin clamp on hepatic glucose production in Par-1b null mice.

**Table 2**

| Par-1b null mice (KO) exhibit increased glucose infusion rate and turnover during hyperinsulinemic-euglycemic clamp relative to wild-type mice (WT) Mice were fed a standard chow diet (4 5% fat) | | | | | | | | |
|---|---|---|---|---|---|---|---|---|
| | Basal Glucose (mg/dl) | Clamp Glucose (mg/dl) | Glucose Infusion Rate (mg/kg/min) | Basal HGP (mg/kg/min) | Clamp HGP (mg/kg/min) | Glucose Turnover (mg/kg/min) | Whole Body Glycolysis (mg/kg/min) | Glycogen Synthesis (mg/kg/min) |
| WT | 105+5 | 109±6 | 451±45 | 166±23 3 | 06±28 8 | 454±2 9 | 291±35 | 173±29 |
| n | 22 | 12 | 9 | 12 | 9 | 12 | 11 | 11 |
| KO | 94±5 | 113±5 | 610±46 | 161±1 9 | -01±45 | 589±56 | 307±41 | 282±44 |
| n | 19 | 10 | 7 | 10 | 7 | 10 | 10 | 10 |
| P | 0 12224 | 0 6878 | 0 0284* | 0 8769 | 0 8954 | 0 0364* | 0 7576 | 00515 |

| | | | | | | | | |
|---|---|---|---|---|---|---|---|---|
| 20 week old male mice were subjected to clamp as described (Kim et al, 2004) Values are stated as mean±SEM Sample number (n) and p values from a student t-test are indicated (*significant) | | | | | | | | |

In order to identify the tissue(s) responsible for the observed increase in insulin sensitivity, we quantified glucose uptake into insulin-responsive tissues during hyperinsulinemic-euglycemic clamp experiments. Insulin-stimulated glucose uptake in epididymal white adipose tissue was increased approximately 4-fold (66+21 nmol/g/min Par-1b^{-/-} versus 17±2 nmol/g/min Par-1b^{+/+}, *P*= 0.0312) (Figure 6A). A trend towards increased brown fat uptake was also observed (Figure 6B). In contrast, insulin-stimulated glucose uptake in skeletal muscle (gastrocnemius) was not significantly different between Par-1b null and WT mice (Figure 6C).

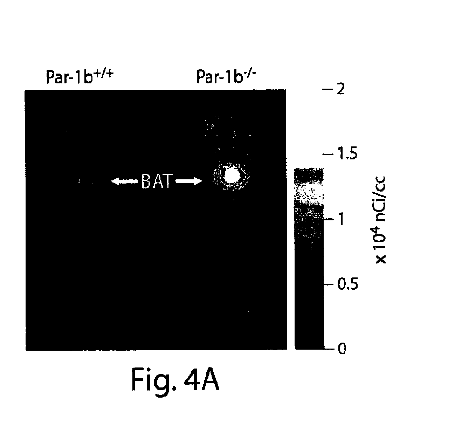

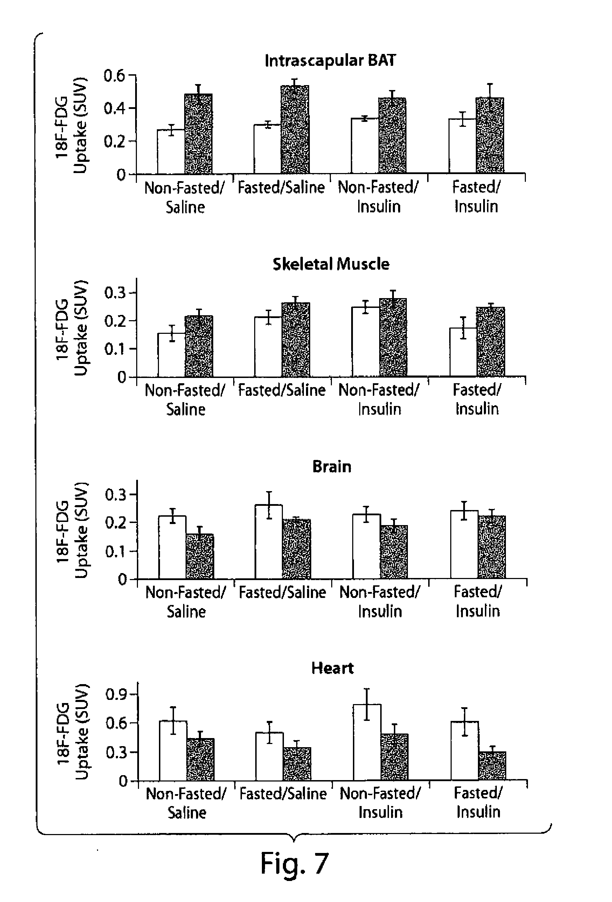

Use of the hyperinsulinemic-euglycemic clamp approach to determine 2-¹⁴C-deoxyglucose uptake indicated a dramatic increase in WAT uptake of Par-1b null mice relative to wild-type mice, but only a trend towards increased uptake in BAT. Since the level of glucose uptake in BAT in all mice tested was high relative to both WAT and skeletal muscle during clamp experiments we reasoned that small increases in BAT uptake in the Par-1b null mice might make a significant contribution to insulin sensitivity. Consequently, we assessed BAT uptake in male mice using an alternative method to either confirm or refute the trend towards increased glucose uptake in BAT of Par-1b null mice during clamp experiments. As seen in Figure 4, ¹⁸F-fluorodeoxyglucose (¹⁸F -FDG) microPET analyses revealed remarkably enhanced uptake in the intrascapular brown adipose tissue (BAT) of Par-1b null mice relative to wild-type mice. Quantitation of glucose uptake into Par-1b null BAT indicated a statistically significant 2-fold increase relative to wild-type mice in both fasting and nonfasting conditions (ANOVA p-value << 0.0001) (Figure 7). This enhancement of glucose uptake into BAT of Par-1b null mice provided additional evidence that adipose tissue plays a significant role in altered glucose metabolism in these mice. In addition, differences observed with microPET imaging were seen in the absence of acute insulin challenge, indicating that there is a constitutive increase in glucose uptake in BAT from Par1b null mice. WAT could not be analyzed by non-invasive microPET imaging especially in the lean Par-1b null mice and thus, WAT was not included in these analyses. There was a statistically significant increase in glucose uptake in skeletal muscle in Par-1b null mice overall by ANOVA (p = 0.01). However, consistent with hyperinsulinemic-euglycemic clamp experiments, this did not reach statistical significance within individual conditions both in the absence and presence of acute insulin challenge (p ≥ 0.1). In contrast, both brain and heart tissue of Par-1b null mice showed overall reduced basal glucose uptake by microPET (ANOVA p-values; 0.03 and 0.005, respectively). In brain this was independent of insulin challenge or fasting. In fasted heart, glucose uptake was lower in both wild-type and Par-1b null mice compared to nonfasted heart, consistent with conversion to fatty acid metabolism. Par-1b null heart tissue appeared non-responsive to insulin and thus, the reduction in glucose uptake by Par-1b null heart tissue relative to control was accentuated by acute insulin challenge, reaching statistical significance (p = 0.05).

### Analysis of protein expression and insulin signaling in Par-1b null adipocytes.

The observed enhancement in adipocyte-mediated glucose uptake in Par-1b^{-/-} mice might be explained by several potential mechanisms (see discussion). We assessed Par-1b expression in insulin-sensitive tissues using a β-galactosidase reporter driven by the endogenous Par-1b promoter (Figure 8) and Western blot analysis (data not shown). Par-1b is highly expressed in BAT, WAT and liver, with no significant expression in skeletal muscle. Based on this expression pattern, it is plausible that Par-1b regulates glucose uptake via an adipocyte-autonomous mechanism. We monitored the expression of several metabolic regulators from adipose tissues of Par-1b^{-/-} and Par-1b^{+/+} mice. Western blotting analyses did not indicate any consistent differences in UCP1, UCP2, PPARγ or GLUT4 protein levels of BAT (Figure 9) or WAT (data not shown). GLUT1 levels appear to be slightly higher in Par-1b null mice relative to wild-type (Figure 9). We also evaluated the insulin signaling pathway in Par-1b^{-/-} mice by injecting wild-type and null mice intraperitoneally with 0.4 U/kg insulin (Figure 10). We did not detect increased IRS-1 tyrosine phosphorylation, IRS-1 associated PI-3 kinase, AKT activation or GSK-3 activation, suggesting that the proximal insulin signaling pathway is largely unaffected by Par-1b deficiency (Figure 10 and data not shown). Since Par-1b is a member of the AMP activated kinase (AMPK) subfamily, we also monitored phospho-AMPK and phospho-acetyl CoA carboxylase levels, neither or which were altered in the absence of Par-1b (Figure 9 and data not shown).

### Discussion

In this study we show that the Ser/Thr protein kinase Par-1b/MARK2/Emk is a novel regulator of adiposity, insulin sensitivity and glucose metabolism using mice null for Par-1b. Par-1b^{-/-} mice are growth retarded during embryogenesis (13.5 days post coitus onward) and after birth. Postnatally, Par-1b null mice exhibit global reduction in organ size of ~20%, with the exception of white and brown adipose tissue (40% reduction relative to wild-type at 10 weeks of age). These decreases in organ mass and adiposity are concomitant with 25% reductions in serum IGF-1 levels. IGF-1 signaling is critical for embryonic and postnatal growth (34, 35). Mouse models of an IGF-1 receptor hypomorph and liver specific deletion of IGF-1 also exhibit global growth retardation with disproportionate decreases in adipose tissue (36, 37). IGF-1 signaling has also been shown to be important for the proliferation and differentiation of adipocytes *in vitro* (38). Taken together our data suggest that Par-1b is essential for IGF-1-mediated growth and may lead to decreased adiposity due to deficits in IGF-1-driven adipogenesis. In contrast to Par-1b^{-/-} mice, both humans and mice deficient in IGF-1/IGF-1R signaling exhibit insulin resistance. Consequently it is difficult to explain insulin hypersensitivity in Par-1b^{-/-} mice based solely on IGF-1 deficiency. Growth hormone receptor (GHR^{-/-}) deficient mice, like Par-1b nulls, exhibit increased insulin sensitivity and growth retardation. However, unlike Par-1b null mice, GHR^{-/-} mice also display increased adiposity with age, susceptibility to diet-induced obesity, reduced body temperatures and normal embryonic growth (39-41). These data suggest that the Par-1b^{-/-} metabolic phenotypes described here are not likely to be solely a result of GH-mediated effects.

Resistance to weight gain on a high fat diet and hyperphagy concomitant with increased glucose uptake into adipose tissue (WAT and BAT) are indicative of a metabolic uncoupling defect. Although we do not detect changes in the protein levels of UCP1, UCP2 or PPARγ, the activities of these and/or other proteins critical for normal metabolic function may be altered in Par-1b^{-/-} mice. Average body temperatures of Par-1b^{-/-} mice are slightly elevated, suggesting that increased energy consumption is most likely dissipated via increased heat generation. Although a physiologically normal increase in BAT-mediated thermogenesis (due to smaller body size and reduced adiposity) might lead to enhanced glucose uptake in BAT, this appears not to be the case since Par-1b^{-/-} mice do not exhibit reduced body temperatures. Recent studies have shown that perturbations in β-adrenergic activity and subsequent BAT-mediated thermogenesis can regulate diet induced obesity and insulin sensitivity in the mouse (42, 43). These studies suggest that a direct effect of Par-1b on β-adrenergic activity and/or BAT-mediated thermogenesis could explain several of the phenotypes described here.

We did not detect significant increases in IRS-1, AKT or GSK3 phosphorylation in insulin-treated Par-1b^{-/-} adipose tissue, indicating that increased glucose uptake into adipose tissue is not due to proximal insulin signaling enhancement. FDG-microPET imaging of Par-1b^{-/-} mice indicates that enhanced glucose uptake in BAT can be observed after fasting, in the absence of an acute insulin challenge. This finding suggests an insulin independent defect in BAT-mediated glucose uptake. It is possible that Par-1b acts downstream of the activation of AKT in glucose transport. Interestingly, the yeast homologues of Par-1b, kin1 and kin2, have been shown to play a role in vesicular trafficking via interaction with the exocyst complex and it has been suggested that mammalian Par-1b interacts with syntaxin-4 (2). The exocyst and syntaxin-4 have been implicated in GLUT4 transport (44-49). In addition, Par-3/ASIP, which is a direct downstream target of Par-1b, has been shown using overexpression studies to inhibit glucose uptake in 3T3L1 adipocytes (50). Both LKB1 and atypical PKC, two upstream regulators of Par-1, have also been shown to play a role in regulation of glucose metabolism (20, 52, 53). AMPK and Par-1b, which share significant homology within their kinase domains, are part of a larger subfamily of kinases that includes 13 members, most of which appear to be regulated at least in part by LKB 1. Indeed, as our data suggests for Par-1b, AMPK regulates glucose uptake by a phospatidylinositol 3-kinase independent mechanism (54). Par-1b and AMPK also share a nearly identical phosphorylation consensus sequence as determined by peptide library screening (unpublished results, Reuben Shaw, Ben Turk and Lewis Cantley). Arguing against a role for Par-1b in GLUT4 mediated transport is a recent RNAi screen performed in 3T3L1 adipocytes (51). This study indicates that knockdown of either Par-1b/MARK2 or C-TAK1/MARK3 had no significant effect on 2-deoxyglucose uptake in either the basal state or in response to insulin. Assuming the efficacy of RNAi knockdown of these two proteins was sufficient to block their function, this data argues that Par-1b has no direct role in GLUT4-mediated glucose uptake and argues against an adipocyte-autonomous function for Par-1b in glucose uptake. Conclusive determination of the mechanism(s) by which Par-1b regulates glucose uptake will await further analyses, particularly tissue-specific deletion of Par-1b.

The finding that Par-1b regulates glucose metabolism and adiposity presents the exciting possibility that Par-1b kinase inhibitors might be used to improve insulin sensitivity and/or ameliorate obesity. Par-1b should be amenable to targeting by small molecules and inhibition of the kinase might be predicted to improve several symptoms associated with Type 2 diabetes.

### Materials and Methods

**Animal Procedures.** All animal procedures were approved by the Washington University School of Medicine Animal Studies Committee. The generation of Par-1b null mice has been described previously (31). Heterozygous Par-1b mice of the strain background C57B1/6;129X1SvJ were interbred to generate the mice used in this study. Mice encoding β-galactosidase in the Par-1b gene were kindly provided by M. Darmon (30). Unless otherwise noted mice were fed a standard chow diet (Purina Mills, Lab Diet 5053 containing 4.5% fat, 55% carbohydrate, 20% protein, 4.7% fiber).

**Animal weight measurements.** Embryos and pups generated from heterozygous crosses were weighed and genotyping was performed by PCR analysis of tail clippings. Embryos were harvested, rinsed in PBS and weighed using a Mettler AE50 scale (Mettler Toledo AG, New Jersey, USA) at the indicated times. Plug observation was recorded as 0.5 days post coitis (dpc). Post-natal growth curves were obtained by weighing mice at the indicated time points using an Ohaus Scout scale (Ohaus Corp., New Jersey, USA). Mean body weights at each time point were compiled from 5 to 13 embryos or from 4 to 19 mice per genotype.

**Histological analysis.** For histological studies, tissues were fixed in 10% neutral-buffered formalin, rinsed in PBS, and stored in 70% ethanol. Fixed tissues were embedded in paraffin by standard procedures. Blocks were sectioned (5 µm) and stained with hematoxylin and eosin. White adipocyte cell number and size was determined by counting multiple independent fields of view at 30X magnification using an Olympus BX60 microscope. **Tissue dissection and X-gal staining.** Brain, brown adipose, white adipose, heart, soleus muscle, pancreas and liver were dissected from mice of 3-5 month of age. The mPar-1b mice have been described (30). Whole mount tissues were dissected and cut into several 3-6 mm sections, washed with PBS 3 times, fixed at 4°C in PBS containing 1% formaldehyde, 0.2% glutaldehyde, 0.02% NP 40, 1mM MgCl₂. After fixation, tissues were washed 3 times with PBS, stained with 1mg/ml X-gal in DMF, 2mM MgCl₂, 5mM Potassium ferricyanide, 5 mM Potassium ferrocyanide, 100mM D-galactose in PBS at 37°C overnight. The tissues were washed with PBS, post fixed with 4% paraformaldehyde in PBS, processed, paraffinized, and sectioned for microscopy.

**High fat diet studies.** At 4 weeks of age Par-1b KO and WT mice were weighed and their regular rodent chow was replaced with Adjusted Calories Diet (42% fat) from Harlan Teklad (88137). The weight of the high fat chow was recorded and food consumption and mouse weight was determined for individual mice on a weekly basis for 16 weeks.

**Hyperinsulinemic-euglycemic clamp studies.** Male Par-1b null mice and wild type littermates (n=19~22) were studied at the NIH-Yale Mouse Metabolic Phenotyping Center to assess the changes in tissue-specific insulin action and glucose metabolism *in vivo.* After 1 month of acclimatization (followed by 8 weeks of high fat diet administration for the high fat diet studies) at the Yale facility, whole body fat and lean mass were measured in awake mice using ¹H-MRS (Bruker Mini-spec Analyzer, Echo Medical Systems, Houston, TX) as described previously (33). Please see supplementary materials and methods for detailed protocol.

**Metabolic measurements.** To investigate a diverse range of metabolic measurements including activity, food and water consumption, and energy expenditure, comprehensive animal metabolic monitoring system (CLAMS: Columbus instruments, Columbus, OH, USA) was used. During data analysis, energy expenditure, feeding, and drinking are normalized with respect to body weight. Energy expenditure and respiratory quotient (RQ) were calculated from the gas exchange data previously collected: RQ as the ratio of VCO₂ to VO₂, Heat = (3.815 + 1.232 * RQ) * VO₂. Activity was measured on an x and z-axis using infrared beams to count the amount of beam breaks during the specified measurement period. Feeding is measured by recording the difference in the scale measurement of the Center-Feeder from one time point to another. Drinking was measured through a volumetric drinking monitor.

**Glucose and insulin tolerance tests.** Randomly fed or fasted (12 h) mice were analyzed as indicated. Serum insulin levels were determined by radioimmunoassay using rat insulin as a standard and ELISA (R&D Systems, as specified by manufacturer). Blood glucose levels were determined using a B-Glucose Photometer and B-glucose cuvettes (HemoCue AB, Angelholm, Sweden). Insulin tolerance tests (ITT) were performed on fasted (6 hr) 8-10 week old mice. Blood glucose values were measured immediately before and at 15 min intervals after intraperitoneal injection of insulin (0.30 IU/kg HumulinR, Eli Lilly and Company, Indianapolis, USA). Glucose tolerance tests (GTTs) were performed on fasted (12 hr) 12 week old animals. Mice were injected intraperitoneally with D-glucose (20% solution; 1 g/kg of body weight) and blood glucose levels were determined at the indicated times post injection.

**Serum factor quantitation.** Serum levels of insulin, triglycerides, adiponectin, leptin, free fatty acids and cholesterol were determined by Ani Lytics Incorporated (Gaithersburg, MD). Serum IGF-1 (R&D Systems, Minneapolis, MN) and growth hormone (Diagnostic Systems Laboratories, Inc., Webster, TX) levels were measured by ELISA as per the manufacturer's instructions.

**microPET studies.** For microPET imaging, cohorts of 8 week old male wild-type and Par-1b null mice were repetitively imaged once a week for four consecutive weeks, each time under a different condition. Mice were either fasted overnight or allowed access to standard rodent chow (non-fasted). The next morning, either 0.5 IU/kg insulin (Humulin N) or saline was administered to mice by IP injection. Thirty minutes after the IP injection, mice were lightly anesthetized with isoflurane followed by a tail vein injection of ¹⁸F-FDG (≈ 400-500 µCi). Immediately following injection of radiotracer, mice were placed supine within the microPET scanner (R4, Concorde MicroSystems, Knoxville, TN) and imaged (≈10 min acquisition time; 1 bed position; OSEM reconstruction; isotropic image resolution 1.8 mm@ 1 cm, 2.68 mm@2 cm). Mice were allowed to recover, then anesthetized and imaged again at 1 hour and 2 hours post injection of radiotracer. MicroPET images were corrected for decay, but not attenuation or scatter, and stacked regions-of-interest (ROI) of relevant tissues and organs were analyzed with AnalyzePC 6.0 software. Data for accumulation of ¹⁸F-FDG on microPET images were expressed as standard uptake values (SUV), representing counts per gram of tissue divided by injected dose of radioactivity per gram of animal weight (55, 56). Data are presented as mean values ± SEM. Groups were statistically analyzed by one-way NOVA and Student's t-test (Microsoft Excel).

**Western blot analysis.** Anti-UCP1 and anti-UCP2 were obtained from Alpha Diagnostic Intl. Inc. (San Antonio, Tx); anti-GLUT1 anti-GLUT4 were obtained from Abcam (Cambridge, MA); anti-p110α was obtained from BD Biosciences (San Jose, CA); Anti-PPARγ, anti-p85 and anti-IRS-1 antibodies were obtained from Upstate (Lake Placid, NY); anti-phospho-AMPK, total AMPK, phospho-GSK-3, GSK-3α, GSK-3β were obtained from Cell Signaling Techonologies (Danvers, MA); anti-P-Tyr (4G10) was a gift from Dr. Tom Roberts. Tissue lysates were prepared by standard methods as in (31). Please see supplementary Materials and Methods for detailed protocols.

### Supplementary Materials and Methods

**Animal weight measurements.** Embryos and pups generated from heterozygous crosses were weighed and genotyping was performed by PCR analysis of tail clippings. Embryos were harvested, rinsed in PBS and weighed using a Mettler AE50 scale (Mettler Toledo AG, New Jersey, USA) at the indicated times. Plug observation was recorded as 0.5 days post coitis (dpc). Post-natal growth curves were obtained by weighing mice at the indicated time points using an Ohaus Scout scale (Ohaus Corp., New Jersey, USA). Mean body weights at each time point were compiled from 5 to 13 embryos or from 4 to 19 mice per genotype.

**Histological analysis.** For histological studies, tissues were fixed in 10% neutral-buffered formalin, rinsed in PBS, and stored in 70% ethanol. Fixed tissues were embedded in paraffin by standard procedures. Blocks were sectioned (5 µm) and stained with hematoxylin and eosin. White adipocyte cell number and size was determined by counting multiple independent fields of view at 30X magnification using an Olympus BX60 microscope.

**Tissue dissection and X-gal staining.** Brain, brown adipose, white adipose, heart, soleus muscle, pancreas and liver were dissected from mice of 3-5 month of age. The mPar-1b mice have been described (30). Whole mount tissues were dissected and cut into several 3-6 mm sections, washed with PBS 3 times, fixed at 4°C in PBS containing 1% formaldehyde, 0.2% glutaldehyde, 0.02% NP 40, 1mM MgCl₂. After fixation, tissues were washed 3 times with PBS, stained with 1mg/ml X-gal in DMF, 2mM MgCl₂ , 5mM Potassium ferricyanide, 5 mM Potassium ferrocyanide, 100mM D-galactose in PBS at 37°C overnight. The tissues were washed with PBS, post fixed with 4% paraformaldehyde in PBS, processed, paraffinized, and sectioned for microscopy.

**Western blot analysis.** Anti-UCP1 and anti-UCP2 were obtained from Alpha Diagnostic Intl. Inc. (San Antonio, Tx); anti-GLUT1 anti-GLUT4 were obtained from Abcam (Cambridge, MA); anti-p110α was obtained from BD Biosciences (San Jose, CA); Anti-PPARγ, anti-p85 and anti-IRS-1 antibodies were obtained from Upstate (Lake Placid, NY); anti-phospho-AMPK, total AMPK, phospho-GSK-3, GSK-3α, GSK-3β were obtained from Cell Signaling Techonologies (Danvers, MA); anti-P-Tyr (4G10) was a gift from Dr. Tom Roberts. Tissue lysates were prepared by standard methods as in (31). Tissues for immunoblot analyses were prepared by homogenization in lysis buffer containing (50 mM Tris [pH 8.0], 100 mM NaCl, 2 mM dithiothreitol [DTT], 5 mM EDTA, 0.5% NP-40, 1 mM microcystin, 1 mM sodium orthovanadate, 2 mM phenylmethylsulfonyl fluoride, 0.15U of aprotinin per ml, 20 mM leupeptin, 20 mM pepstatin) at 4°C for 20 min. Lysates were clarified by centrifugation, and protein concentrations were determined using the Bio-Rad protein assay kit. For Western blot analysis, 50 µg of total protein was resolved by sodium dodecyl sulfate-polyacrylamide gel electrophoresis (SDS-PAGE) (7% polyacrylamide). Immunoprecipiation of IRS-1 was performed by incubating 1 ug of anti-IRS-1 antibody with 1 mg of total tissue lysate for 2 hours followed by a 1 hour incubation with protein G Sepharose at 4°C.

**Glucose and insulin tolerance tests.** Randomly fed or fasted (12 h) mice were analyzed as indicated. Serum insulin levels were determined by radioimmunoassay using rat insulin as a standard and ELISA (R&D Systems, as specified by manufacturer). Blood glucose levels were determined using a B-Glucose Photometer and B-glucose cuvettes (HemoCue AB, Angelholm, Sweden). Insulin tolerance tests (ITT) were performed on fasted (6 hr) 8-10 week old mice. Blood glucose values were measured immediately before and at 15 min intervals after intraperitoneal injection of insulin (0.30 IU/kg HumulinR, Eli Lilly and Company, Indianapolis, USA). Glucose tolerance tests (GTTs) were performed on fasted (12 hr) 12 week old animals. Mice were injected intraperitoneally with D-glucose (20% solution; 1 g/kg of body weight) and blood glucose levels were determined at the indicated times post injection.

**Serum factor quantitation.** Serum levels of insulin, triglycerides, adiponectin, leptin, free fatty acids and cholesterol were determined by Ani Lytics Incorporated (Gaithersburg, MD). Serum IGF-1 (R&D Systems, Minneapolis, MN) and growth hormone (Diagnostic Systems Laboratories, Inc., Webster, TX) levels were measured by ELISA as per the manufacturer's instructions.

**Hyperinulinemic-Euglycemic clamp studies.** At least 4 days before the metabolic experiments, mice were anesthetized, and an indwelling catheter was inserted in the right internal jugular vein as previously described (33). All procedures were approved by the Yale University Animal Care and Use Committee. After overnight fast, a 2-hour hyperinsulinemic-euglycemic clamp was conducted in awake Par-1b null mice and wild-type littermates. Briefly, insulin (HumulinR insulin, Eli Lilly, Indianapolis, IN) was primed (150mU/kg body weight) and continuously infused at 15 pmol/kg/min, and 20% glucose was infused at variable rates to maintain glucose at basal concentrations. Basal and insulin-stimulated whole body glucose turnover was estimated with a continuous infusion of (3-³H) glucose (PerkinElmer Life and Analytical Sciences, Ind.) for 2 hours prior to the clamps (0.05 µCi/min) and throughout the clamps (0.1 µCi/min), respectively. To estimate insulin-stimulated glucose uptake in individual tissues, 2-deoxy-D- [1-¹⁴C] glucose (2-[¹⁴C] DG) was administered as a bolus (10 µCi) at 75 min after the start of clamps. At the end of clamps, mice were anesthetized, and tissues were taken for biochemical analysis (33). Rates of basal hepatic glucose production (HGP) and insulin-stimulated whole body glucose uptake were determined as the ratio of the [³H] glucose infusion rate (disintegrations per minute; dpm/min) to the specific activity of plasma glucose (dpm/µmol) at the end of basal period and during the final 30 min of clamps, respectively. Insulin-stimulated rates of HGP during clamps were determined by subtracting the glucose infusion rate from the whole body glucose uptake. Whole body glycolysis and glycogen plus lipid synthesis from glucose were estimated as previously described (33). Since 2-deoxyglucose is a glucose analog that is phosphorylated but not further metabolized, insulin-stimulated glucose uptake in individual tissues can be estimated by determining the tissue content of 2-[¹⁴C] DG-6-P. Based on this, glucose uptake in individual tissues was calculated from plasma 2-[¹⁴C] DG profile and tissue 2-[¹⁴C] DG-6-P content as previously described (33). Two independent sets of experiments were performed. Mice used in the first experiment were 5 to 6 months old whereas mice used in the second experiment were 4 months old.

**Hyperinsulinemic-euglycemic clamp studies.** Male Par-1b null mice and wild type littermates (n=19~22) were studied at the NIH-Yale Mouse Metabolic Phenotyping Center to assess the changes in tissue-specific insulin action and glucose metabolism *in vivo.* After 1 month of acclimatization (followed by 8 weeks of high fat diet administration for the high fat diet studies) at the Yale facility, whole body fat and lean mass were measured in awake mice using ¹H-MRS (Bruker Mini-spec Analyzer, Echo Medical Systems, Houston, TX) as described previously (33).

**Metabolic measurements.** To investigate a diverse range of metabolic measurements including activity, food and water consumption, and energy expenditure, comprehensive animal metabolic monitoring system (CLAMS: Columbus instruments, Columbus, OH, USA) was used. During data analysis, energy expenditure, feeding, and drinking are normalized with respect to body weight. Energy expenditure and respiratory quotient (RQ) were calculated from the gas exchange data previously collected: RQ as the ratio of VCO₂ to VO₂, Heat = (3.815 + 1.232 * RQ) * VO₂. Activity was measured on an x and z-axis using infrared beams to count the amount of beam breaks during the specified measurement period. Feeding is measured by recording the difference in the scale measurement of the Center-Feeder from one time point to another. Drinking was measured through a volumetric drinking monitor.

### References

1. Guo, S. & Kemphues, K. J. (1995) Cell 81, 611-620.
2. Elbert, M., Rossi, G. & Brennwald, P. (2005) Mol Biol Cell 16, 532-49.
3. Drewes, G. & Nurse, P. (2003) FEBS Lett 554, 45-9.
4. Cox, D. N., Lu, B., Sun, T. Q., Williams, L. T. & Jan, Y. N. (2001) Curr Biol 11, 75-87.
5. Huynh, J. R., Petronczki, M., Knoblich, J. A. & St Johnston, D. (2001) Curr Biol 11, 901-6.
6. Doerflinger, H., Benton, R., Torres, I. L., Zwart, M. F. & St Johnston, D. (2006) Curr Biol 16, 1090-5.
7. Bayraktar, J., Zygmunt, D. & Carthew, R. W. (2006) J Cell Sci 119, 711-21.
8. Ossipova, O., Dhawan, S., Sokol, S. & Green, J. B. (2005) Dev Cell 8, 829-41.
9. Bohm, H., Brinkman, V., Drab, M., Henske, A. & Kurzchalia, T. V. (1997) Curr. Biol. 7, 603-606.
10. Biernat, J., Wu, Y. Z., Timm, T., Zheng-Fischhofer, Q., Mandelkow, E., Meijer, L. & Mandelkow, E. M. (2002) Mol Biol Cell 13, 4013-28.
11. Cohen, D., Brennwald, P. J., Rodriguez-Boulan, E. & Musch, A. (2004) J Cell Biol 164, 717-27.
12. Cohen, D., Rodriguez-Boulan, E. & Musch, A. (2004) Proc Natl Acad Sci U S A 101, 13792-7.
13. Chen, Y. M., Wang, Q. J., Hu, H. S., Yu, P. C., Zhu, J., Drewes, G., Piwnica-Worms, H. & Luo, Z. G. (2006) Proc Natl Acad Sci U S A 103, 8534-9.
14. Drewes, G., Trinczek, B., Illenberger, S., Biernat, J., Schmitt-Ulms, G., Meyer, H. E., Mandelkow, E. M. & Mandelkow, E. (1995) J Biol Chem 270, 7679-88.
15. Drewes, G., Ebneth, A., Preuss, U., Mandelkow, E.-M. & Mandelkow, E. (1997) Cell 89, 297-308.
16. Nishimura, I., Yang, Y. & Lu, B. (2004) Cell 116, 671-82.
17. Doerflinger, H., Benton, R., Shulman, J. M. & St Johnston, D. (2003) Development 130, 3965-75.
18. Sun, T. Q., Lu, B., Feng, J. J., Reinhard, C., Jan, Y. N., Fantl, W. J. & Williams, L. T. (2001) Nat Cell Biol 3, 628-36.
19. Ducharme, N. A., Hales, C. M., Lapierre, L. A., Ham, A. J., Oztan, A., Apodaca, G. & Goldenring, J. R. (2006) Mol Biol Cell 17, 3625-37.
20. Lizcano, J. M., Goransson, O., Toth, R., Deak, M., Morrice, N. A., Boudeau, J., Hawley, S. A., Udd, L., Makela, T. P., Hardie, D. G. & Alessi, D. R. (2004) Embo J 23, 833-43.
21. Alessi, D. R., Sakamoto, K. & Bayascas, J. R. (2006) Annu Rev Biochem 75, 137-63.
22. Hurov, J. B., Watkins, J. L. & Piwnica-Worms, H. (2004) Curr Biol 14, 736-41.
23. Suzuki, A., Hirata, M., Kamimura, K., Maniwa, R., Yamanaka, T., Mizuno, K., Kishikawa, M., Hirose, H., Amano, Y., Izumi, N., Miwa, Y. & Ohno, S. (2004) Curr Biol 14, 1425-35.
24. Vaccari, T., Rabouille, C. & Ephrussi, A. (2005) Curr Biol 15, 255-61.
25. Kusakabe, M. & Nishida, E. (2004) Embo J 23, 4190-201.
26. Inglis, J. D., Lee, M. & Hill, R. E. (1993) Mammalian Genome 4, 401-403.
27. Peng, C.-Y., Graves, P. R., Ogg, S., Thoma, R. S., Byrnes, M. J., Wu, Z., Stephenson, M. & Piwnica-Worms, H. (1998) Cell Growth Differ. 9, 197-208.
28. Kato, T., Satoh, S., Okabe, H., Kitahara, O., Ono, K., Kihara, C., Tanaka, T., Tsunoda, T., Yamaoka, Y., Nakamura, Y. & Furukawa, Y. (2001) Neoplasia 3, 4-9.
29. Trinczek, B., Brajenovic, M., Ebneth, A. & Drewes, G. (2004) J Biol Chem 279, 5915-23.
30. Bessone, S., Vidal, F., Le Bouc, Y., Epelbaum, J., Bluet-Pajot, M.-T. & Darmon, M. (1999) Developmental Biology 214, 87-101.
31. Hurov, J. B., Stappenbeck, T. S., Zmasek, C. M., White, L. S., Ranganath, S. H., Russell, J. H., Chan, A. C., Murphy, K. M. & Piwnica-Worms, H. (2001) Mol Cell Biol 21, 3206-19.
32. Bjorbaek, C. & Kahn, B. B. (2004) Recent Prog Horm Res 59, 305-31.
33. Kim, J. K., Fillmore, J. J., Sunshine, M. J., Albrecht, B., Higashimori, T., Kim, D. W., Liu, Z. X., Soos, T. J., Cline, G. W., O'Brien, W. R., Littman, D. R. & Shulman, G. I. (2004) J Clin Invest 114, 823-7.
34. Baker, J., Liu, J. P., Robertson, E. J. & Efstratiadis, A. (1993) Cell 75, 73-82.
35. Liu, J. P., Baker, J., Perkins, A. S., Robertson, E. J. & Efstratiadis, A. (1993) Cell 75, 59-72.
36. Holzenberger, M., Hamard, G., Zaoui, R., Leneuve, P., Ducos, B., Beccavin, C., Perin, L. & Le Bouc, Y. (2001) Endocrinology 142, 4469-78.
37. Sjogren, K., Jansson, J. O., Isaksson, O. G. & Ohlsson, C. (2002) Endocrine 19, 249-56.
38. Gregoire, F. M., Smas, C. M. & Sul, H. S. (1998) Physiol Rev 78, 783-809.
39. Bartke, A. (2005) Endocrinology 146, 3718-23.
40. Berryman, D. E., List, E. O., Kohn, D. T., Coschigano, K. T., Seeley, R. J. & Kopchick, J. J. (2006) Endocrinology 147, 2801-8.
41. Hauck, S. J., Hunter, W. S., Danilovich, N., Kopchick, J. J. & Bartke, A. (2001) Exp Biol Med (Maywood) 226, 552-8.
42. Bachman, E. S., Dhillon, H., Zhang, C. Y., Cinti, S., Bianco, A. C., Kobilka, B. K. & Lowell, B. B. (2002) Science 297, 843-5.
43. Kim, H., Pennisi, P. A., Gavrilova, O., Pack, S., Jou, W., Setser-Portas, J., East-Palmer, J., Tang, Y., Manganiello, V. C. & Leroith, D. (2006) Am J Physiol Endocrinol Metab 290, E1227-36.
44. Ewart, M. A., Clarke, M., Kane, S., Chamberlain, L. H. & Gould, G. W. (2005) J Biol Chem 280, 3812-6.
45. Inoue, M., Chang, L., Hwang, J., Chiang, S. H. & Saltiel, A. R. (2003) Nature 422, 629-33.
46. Spurlin, B. A., Park, S. Y., Nevins, A. K., Kim, J. K. & Thurmond, D. C. (2004) Diabetes 53, 2223-31.
47. Yang, C., Coker, K. J., Kim, J. K., Mora, S., Thurmond, D. C., Davis, A. C., Yang, B., Williamson, R. A., Shulman, G. I. & Pessin, J. E. (2001) J Clin Invest 107, 1311-8.
48. Zeigerer, A., Lampson, M. A., Karylowski, O., Sabatini, D. D., Adesnik, M., Ren, M. & McGraw, T. E. (2002) Mol Biol Cell 13, 2421-35*.*
49. Kessler, A., Tomas, E., Immler, D., Meyer, H. E., Zorzano, A. & Eckel, J. (2000) Diabetologia 43, 1518-27.
50. Kotani, K., Ogawa, W., Hashiramoto, M., Onishi, T., Ohno, S. & Kasuga, M. (2000) J Biol Chem 275, 26390-5.
51. Tang, X., Guilherme, A., Chakladar, A., Powelka, A. M., Konda, S., Virbasius, J. V., Nicoloro, S. M., Straubhaar, J. & Czech, M. P. (2006) Proc Natl Acad Sci U S A 103, 2087-92.
52. Shaw, R. J., Lamia, K. A., Vasquez, D., Koo, S. H., Bardeesy, N., Depinho, R. A., Montminy, M. & Cantley, L. C. (2005) Science 310, 1642-6.
53. Farese, R. V., Sajan, M. P. & Standaert, M. L. (2005) Biochem Soc Trans 33, 350-3.
54. Long, Y. C. & Zierath, J. R. (2006) J Clin Invest 116, 1776-83.
55. Luker, G., Sharma, V., Pica, C., Dahlheimer, J., Li, W., Ochesky, J., Ryan, C., Piwnica-Worms, H. & Piwnica-Worms, D. (2002) Proc Natl Acad Sci USA 99, 6961-6966.
56. Luker, G. D., Sharma, V. & Piwnica-Worms, D. (2003) Methods 29, 110-22.

### Example 2: High throughput cell-based assay to identify inhibitors of Par-1b

A high throughput cell-based assay is performed by screening a cell line using a phospho-specific antibody for phospho-tau, phospho-Dv1, phospho-Par-3, phospho-Cdc25C, phospho-MAP2 or phospho-MAP4 based on the identification of these proteins as substrates for Par-1b (see Drewes et al., Cell. 1997 Apr 18;89(2):297-308; Ebneth et al., Cell Motil Cytoskeleton. 1999 Nov;44(3):209-24). Such antibodies are commercially available, e.g., from Upstate Biotechnology. Candidate inhibitor compounds are added to cells (e.g. HEK293 cells, CHO cells, NIH3T3 fibroblasts or 3T3L1 adipocytes) in 96 or 384 well plates, and then the treated cells are screened by immunocytochemistry for phosphorylated protein. Candidate inhibitor compounds that inhibit Par-1b kinase activity directly or by affecting a protein upstream in the pathway are those that diminish the phosphorylated protein signal. Whether such compounds directly bind and inhibit Par-1b is confirmed, for example, using an *in vitro* assay as described in Example 3.

### Example 3: High throughput in vitro assay to identify inhibitors of Par-1b

A more direct assay is to screen directly for Par-1b inhibitors *in vitro.* This is a biochemical assay in which kinase activity is assayed. A variety of detectable molecules for determining the kinase activity of Par-1b are available. The kinase assay described in Elbert et al. Mol Biol Cell. 2006 Aug;17:3345-3355 to assay *in vitro* the phosphorylation of Dvl protein by Par-1b may be used.

Alternatively, to increase the quantitative and high throughput nature without needing radioactivity, one can use fluorescence polarization to quantify the amount of activity of the kinase of interest (e.g. Par-1b). A fluorescein-conjugated peptide substrate for Par-1b (such those derived from its optimal consensus sequence in the tau, Dvl, Par-3, Cdc25C, MAP2 and MAP4 proteins) is mixed with Par-1b with or without inhibitor to form an assay mixture. In this assay, a fluorescently labeled peptide substrate is phosphorylated by the kinase and captured on metal-derivatized nanoparticles ("IMAP", Molecular Devices Corporation, Sunnyvale, CA). At high salt concentration, trivalent metal cations bind and capture phosphorylated peptides. The amount of fluoresceinated, phosphorylated peptide bound is measured by an increase in the fluorescence polarization signal caused by a decrease in the molecular mobility of the bound product. (Turek-etienne, et al. (2003) Assay Drug Dev Technol., Aug;1(4):545-53).

It is preferable to determine that candidate inhibitors are specific for Par-1b and not related kinases. To test for specificity, the assays can be repeated (or run in parallel) with additional kinases, such as those in the Par-1 family of kinases. Specificity need not mean that the compound has activity on Par-1b and no activity on other kinases. For example a compound that inhibits Par-1b with a lower IC50 than it inhibits other kinases has sufficient specificity to be effective in specifically reducing Par-1b kinase activity. Similar assays may be performed for the four different isoforms of Par-1b known in the art (isoforms a, b, c, d).

Other aspects of the invention will be clear to the skilled artisan and need not be repeated here. Each reference cited herein is incorporated by reference in its entirety.

The terms and expressions that have been employed are used as terms of description and not of limitation, and there is no intention in the use of such terms and expressions of excluding any equivalents of the features shown and described or portions thereof, it being recognized that various modifications are possible within the scope of the invention.

The present invention will now be described further with reference to the following numbered clauses:-
1. A method for identifying compounds or compositions useful as pharmacological agents for the treatment of diabetes or obesity, comprising:
   contacting a Par-1b polypeptide with a candidate pharmacological agent, and
   determining the kinase activity of the contacted Par-1b polypeptide, wherein a decrease in the kinase activity of the Par-1b polypeptide contacted with the candidate pharmacological agent relative to a control amount of kinase activity of the Par-1b polypeptide is an indication that the candidate pharmacological agent is useful in the treatment of diabetes or obesity.
2. The method of clause 1, further comprising determining a second amount of kinase activity of the Par-1b polypeptide in the absence of the candidate pharmacological agent, and using the second amount of activity of the Par-1b polypeptide as the control amount of activity.
3. The method of clause 1 or 2, wherein the kinase activity of the Par-1b polypeptide is measured by phosphorylation of a tau, Dvl, Par-3, Cdc25C, MAP2 or MAP4 protein or a peptide comprising the phosphorylation site of a tau, Dvl, Par-3, Cdc25C, MAP2 or MAP4 protein.
4. The method of clause 3, wherein the phosphorylation is measured by a phospho-specific antibody or an antigen-binding fragment thereof.
5. The method of clause 1, wherein the Par-1b polypeptide is contained within a cell, and the cell is contacted with the candidate pharmacological agent.
6. A method for treating a subject having or suspected of having disorder characterized by reduced insulin responsiveness or obesity comprising:
   administering to a subject in need of such treatment an effective amount of a compound or composition that decreases the kinase activity of Par-1b polypeptide in the subject, as a treatment for the disorder.
7. The method of clause 6, wherein the disorder characterized by reduced insulin responsiveness is type 2 diabetes.
8. The method of clause 6, wherein the compound or composition decreases the amount of Par-1b polypeptide.
9. The method of clause 8, wherein the compound or composition that decreases the amount of Par-1b polypeptide is a siRNA/RNAi molecule or an antisense nucleic acid molecule.
10. The method of clause 9, wherein the siRNA/RNAi molecule comprises the nucleotide sequence AAG ACU CAA CUG AAC UCC UCC (SEQ ID NO:1).
11. A method for preparing a drug for the treatment of a disorder characterized by reduced insulin responsiveness or obesity, comprising:
   identifying a compound or composition that decreases kinase activity of a Par-1b polypeptide and
   formulating the compound or composition for administration to a subject in need of such treatment.
12. The method of clause 11, wherein the disorder characterized by reduced insulin responsiveness is type 2 diabetes.
13. The method of clause 11, wherein the compound or composition that decreases kinase activity of a Par-1b polypeptide is identified by the method of any of clauses 1-5.

## Claims

1. A method for diagnosing a disorder associated with increased or excessive Par-1b activity comprising
assaying a biological sample obtained from a subject, for a level of expression of Par-1b nucleic acid molecules, a level of expression of Par-1b polypeptide molecules, and/or a level of kinase activity of a Par-1b polypeptide.

2. A method for monitoring the onset, progression, or regression of a disorder associated with increased or excessive Par-1b activity in a subject comprising
assaying biological samples, obtained at sequential times from a subject, for a level of expression of Par-1b nucleic acid molecules, a level of expression of Par-1b polypeptide molecules, and/or a level of kinase activity of a Par-1b polypeptide.

3. The method of claim 1 or claim 2, wherein the level of expression of Par-1b nucleic acid molecules, the level of expression of Par-1b polypeptide molecules, and/or the level of kinase activity of a Par-1b polypeptide is determined relative to a control sample.

4. The method of claim 3, wherein the control sample is a biological sample obtained from a subject who does not to have a disorder associated with increased or excessive Par-1b activity.

5. The method of any of claims 1-4, wherein level of kinase activity of a Par-1b polypeptide is the level or amount of catalytic activity of a Par-1b polypeptide.

6. The method of claim 5, wherein the level of kinase activity of a Par-1b polypeptide is assayed by measuring the level or amount of specific phosphorylation of a Par-1b substrate.

7. The method of claim 6, wherein the Par-1b substrate is tau, Dvl, Par-3, Cdc25C, MAP2 or MAP4 protein or a peptide comprising the phosphorylation site of a tau, Dvl, Par-3, Cdc25C, MAP2 or MAP4 protein.

8. The method of claim 6 or claim 7, wherein the level or amount of specific phosphorylation of the Par-1b substrate is assayed using a phospho-specific antibody or an antigen-binding fragment thereof.

9. The method of any of claims 1-4, wherein the level of expression of Par-1b nucleic acid molecules is assayed by direct RNA amplification, reverse transcription of RNA to cDNA, amplification of cDNA, RT-PCR, real-time RT-PCR, generation of radiolabeled nucleic acids, or Northern blotting.

10. The method of any of claims 1-4, wherein the level of expression of Par-1b polypeptide molecules is assayed using an antibody that specifically binds to Par-1b polypeptide, or an antigen-binding fragment thereof.

11. The method of any of claims 1-10, wherein the biological sample comprises tissue, cells, body fluid, blood, or lymph node fluid.

12. The method of claim 11, wherein the body fluid sample comprises cells and fluid.
